# EUROPÄISCHE PATENTSCHRIFT

(11) **EP 2 919 740 B1**
(45) Veröffentlichungstag und Bekanntmachung des Hinweises auf die Patenterteilung: **07.03.2018**
(21) Anmeldenummer: 13805729.4
(22) Anmeldetag: 12.11.2013
(51) Int. Cl.: A61J 1/03, B32B 27/00

(54) **VORRICHTUNG UND VERFAHREN ZUR HERSTELLUNG, DOSIERUNG UND VERPACKUNG VON ARZNEIMITTELN**
DEVICE AND METHOD FOR PRODUCING, DOSING AND PACKAGING MEDICAMENTS
DISPOSITIF ET PROCÉDÉ DE PRODUCTION, DE DOSAGE ET D'EMBALLAGE DE MÉDICAMENTS

(30) Priorität: 15.11.2012 AT 505162012
(43) Veröffentlichungstag der Anmeldung: 23.09.2015
(73) Patentinhaber: AIT Austrian Institute of Technology GmbH, 1220 Wien (AT)
(72) Erfinder: SCHREIER, Günter, A-8010 Graz (AT); MODRE-OSPRIAN, Robert, A-8563 Ligist (AT)
(74) Vertreter: Wildhack & Jellinek
(86) Internationale Anmeldenummer: PCT/AT2013/050217
(87) Internationale Veröffentlichungsnummer: WO 2014/075119

(56) Entgegenhaltungen:
- WO-A1-2006/007867
- WO-A1-2007/070902
- WO-A1-2009/018922
- WO-A1-2013/110107
- WO-A2-2009/000425

## Beschreibung

Die Erfindung betrifft ein Verfahren zur Erstellung von Arzneimitteln gemäß dem Oberbegriff des Patentanspruchs 1. Weiters betrifft die Erfindung eine Kombination mit einem Arzneimittelblister und einem Speicher gemäß dem Oberbegriff des Patentanspruchs 10. Schließlich betrifft die Erfindung eine Vorrichtung zur Herstellung von Arzneimitteln gemäß dem Oberbegriff des Patentanspruchs 15. Ferner betrifft die Erfindung eine Vorrichtung zur Durchführung einer Therapie gemäß dem Oberbegriff des Patentanspruchs 21.

Aus der Veröffentlichung WO 2009/000425 A2 ist ein System zur Identifizierung und Verifizierung der Echtheit einer Durchdrückpackung und zur Unterstützung der Kontrolle einer Medikamenteneinnahme durch einen Patienten offenbart. Dieses System zeigt eine Durchdrückpackung mit einem Blisterbodenteil mit wenigstens einem auf der Bodenfolie herausgeformten, eine Tablette oder dergleichen Medikamentenportion enthaltenden Behältnis und einer gegen das Blisterbodenteil gesiegelten, durchdrückbaren Deckfolie. Weiters weist das System einen an der Durchdrückpackung angeordneten RFID-Tag mit RFID-Chip und einer RFID-Antenne auf sowie einen dem Patienten zugeordneten RFID-Leser.

Die Erfindung sowie einzelne erfindungsgemäße Verfahren, Kombinationen und Vorrichtungen werden auf dem Gebiet der Analyse der Wirksamkeit sowie im Gebiet der Compliance- oder Adhärenz-Messungen von Arzneimitteln oder Behandlungen eingesetzt.

Ausgangspunkt der Erfindung ist ein Verfahren zur Bestimmung der Empfindlichkeit einer therapeutischen Messgröße einer Person auf Veränderung einer therapeutischen Stellgröße während der Therapie. Die Behandlungsintensität entspricht - in Analogie zur Regelungstechnik - der jeweiligen Stellgröße, der einzustellende physiologische Wert, beispielsweise der Blutdruckwert, entspricht der zu regelnden Messgröße.

In der medizinischen Therapeutik ist es üblich, mit Hilfe von therapeutischen Stellgrößen Einfluss auf unterschiedliche physiologische Messgrößen des Patienten zu nehmen. Beispielsweise werden den Patienten Medikamente in einer bestimmten Dosis - dies ist die Stellgröße - verschrieben, mit denen der Blutdruck - dies ist die Messgröße - beeinflusst wird. Der Grad der Beeinflussung der Messgröße kann kontinuierlich oder punktuell, invasiv oder nicht invasiv mit Hilfe geeigneter Methoden gemessen werden, z.B. der systolische und diastolische Blutdruck mittels eines elektronischen Blutdruckmessgerätes.

Unter der Annahme, dass alle anderen Einflussfaktoren konstant bleiben, kann dabei davon ausgegangen werden, dass der Zusammenhang zwischen Stellgröße und Messgröße m als "Stellgrößen-Messgrößen-Kurve" dargestellt werden kann. Im Bereich der Pharmazie wird dieser Zusammenhang Dosis-Wirkungskurve genannt. Generalisierend für alle therapeutischen Stellgrößen, die in diesem Zusammenhang verwendet werden, steht auch der Begriff Behandlungsintensität.

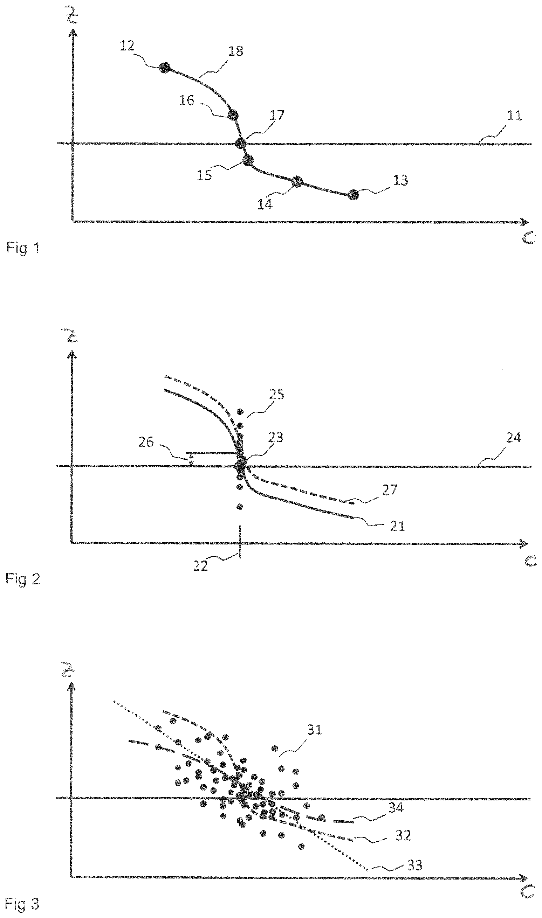

Der Stand der Technik wird anhand zweier gängiger Verfahren näher erläutert. Fig. 1 zeigt eine aus einem ersten Verfahren ermittelte therapeutische Stellgrößen-Messgrößen-Kurve. **Fig. 2** zeigt eine aus einem zweiten Verfahren ermittelte therapeutische Stellgrößen-Messgrößen-Kurve.

In **Fig. 1** ist beispielhaft dargestellt, wie eine Stellgrößen-Messgrößen-Kurve im Zuge einer Behandlung bestimmt werden kann. Wenn z.B. ein Patient auf ein neues blutdrucksenkendes Medikament bzw. Arzneimittel eingestellt wird, soll ein bestimmter Sollwert der Messgröße z, im vorliegenden Fall ein bestimmter Blutdruckwert, erreicht werden. Dazu wählt der Arzt eine initiale Dosis des Medikamentes als therapeutische Stellgröße c und misst, gegebenenfalls zeitversetzt nach einer Einlaufphase, den daraus resultierenden Blutdruck als physiologische Messgröße z. Daraus kann der erste Punkt der Stellgrößen-Messgrößen-Kurve 12 abgeleitet werden. Je nach initialem Wert der Messgröße z wird dann die Behandlungsintensität bzw. die Stellgröße c angepasst und erneut die Messgröße z gemessen. Dieser Vorgang wird wiederholt und es werden für weitere Stellgrößen Messgrößen z; 14, 15, 16 ermittelt, bis jener Wert der Stellgröße gefunden wurde, der zum gewünschten Wert der Messgröße z führt. Somit wird also die Dosis c des Medikamentes gefunden, bei welcher für den jeweiligen Patienten der gewünschte Blutdruck z erreicht wird. Dieser gewünschte Wert wird in Analogie zur Regelungstechnik auch Arbeitspunkt 17 genannt. Durch Verbinden der Punkte, deren jeweilige Koordinatenwerte der Stellgröße c bzw. Messgröße z entsprechen, zu einem Graphen erhält man, wie in **Fig. 1** dargestellt, die Stellgrößen-Messgrößen-Kurve 18.

Im Zuge dieses Einstellvorganges werden daher einzelne Punkte der Stellgrößen-Messgrößen-Kurve bestimmt. Die Messgrößen z bei unterschiedlichen Stellgrößen werden miteinander verglichen und auch die Empfindlichkeit der Messgröße z auf Veränderungen der Stellgröße kann berücksichtigt werden. Die Empfindlichkeit der Messgröße z auf Veränderungen der Stellgröße c entspricht der Steigung der Stellgrößen-Messgrößen-Kurve 18. Jeder Wert der Stellgröße c wird dabei einmal eingestellt und durch Erhöhen bzw. Verringern der Stellgröße c wird mit jeder Messung ein neuer Messpunkt auf der Stellgrößen-Messgrößen-Kurve 18 gefunden.

In einem Szenario bei der Insulin-Therapie von Diabetikern wird beim Einstellvorgang eine individuelle Stellgrößen-Messgrößen-Kurve bestimmt. Während der Therapie misst der Patient zu vorgegebenen Zeitpunkten seinen Blutzuckergehalt als Messgröße z und spritzt anschließend jene Menge c an Insulin, die laut dem aus der persönlicher Erfahrung und ärztlichem Wissen gewonnenen Zusammenhang zwischen Stellgröße und Messgröße z notwendig ist, um den gewünschten Wert der Messgröße z zu erreichen. Mit jeder Medikamenten-Einnahme und zugehörigen Blutzucker-Messung wird demnach auch noch während der laufenden Therapie ein Punkt in der Stellgrößen-Messgrößen-Kurve bestimmt - die Kurve kann dadurch fortwährend an etwaige Veränderungen angepasst werden.

Ein solches Vorgehen kann jedoch gerade bei der Messung der Empfindlichkeit einer Person hinsichtlich Änderungen der Dosierung von Insulin zu gefährlichen Effekten führen, da bereits geringe Unter- oder Überdosierungen schwere Komplikationen verursachen können.

Üblich ist es daher, wie in **Fig. 2** dargestellt, eine Medikation nach anfänglicher Einstellung nicht mehr zu ändern und mögliche Gefährdungen des Patienten zu unterlassen. Sobald der adäquate Wert der Stellgröße 22 sowie die zugehörige initiale Stellgrößen-Messgrößen-Kurve 21 gefunden wurde, wird die Medikation des Patienten für unter Umständen längere Zeit nicht mehr geändert und der Wert der Stellgröße beibehalten.

Wäre die Stellgrößen-Messgrößen-Kurve zeitlich invariant und von weiteren Faktoren nicht beeinflusst, würde aufgrund der konstanten Stellgröße 22 bei jeder einzelnen Messung der Messgröße z ein und derselbe, gewünschte Wert 24 gemessen werden und alle Stellgrößen-Messgrößen-Paare lägen im Arbeitspunkt 23. Aus mehreren Gründen variiert die Messgröße z aber auch bei konstanter Stellgröße: Im Allgemeinen beeinflussen auch Störgrößen die Messgröße z, z.B. Tageszeit, Ernährung, äußere Faktoren, körperliche Aktivität, etc. Zusätzlich ist die Messung oft mit Messungenauigkeiten behaftet. Als Folge werden daher im Arbeitspunkt zu verschiedenen Zeitpunkten vom gewünschten Messgrößenwert abweichende Werte der Messgröße z gemessen werden und es entsteht eine "eindimensionale Punktwolke" 25, die Punkte mit jeweils identischer Stellgröße aufweist.

Unter der Voraussetzung, dass sich die Stellgrößen-Messgrößen-Kurve mit der Zeit nicht verändert, und dass Störgrößen und Messfehler stochastisch verteilt sind, wird der mittlere gemessene Wert der Messgröße z weiterhin der gewünschte Wert der Messgröße z sein. Ändert sich aber die Stellgrößen-Messgrößen-Kurve mit der Zeit - z.B. weil sich der Körper des Patienten an ein verabreichtes Medikament gewöhnt und die Wirksamkeit des Medikamentes mit der Zeit nachlässt - verschiebt sich der Mittelwert der gemessenen Messgrößen z in der Folge aus dem Arbeitspunkt heraus.

Wenn festgestellt wird, dass z.B. aufgrund dieses Gewöhnungseffektes die Medikation des Patienten verändert und somit der Arbeitspunkt verschoben werden muss, sind in der Regel keine Informationen darüber vorhanden, inwiefern sich die Stellgrößen-Messgrößen-Kurve inzwischen geändert hat.

Im Allgemeinen kann keine gesicherte Aussage über die veränderte Kurve getroffen werden und darüber, wie sehr sich eine geringfügige Änderung der Stellgrößen-Messgrößen-Kurve - aus dem Arbeitspunkt heraus - auf die Messgröße z auswirken könnte, d.h. welche Steigung die Stellgrößen-Messgrößen-Kurve im Arbeitspunkt hat.

Die Aufgabe der Erfindung liegt darin, geeignete Arzneimittel oder Vorrichtungen zur Verfügung zu stellen, mit denen eine Messung der individuellen Dosis-Wirkungskurve eines Patienten ermöglicht wird, wobei laufend die Dosis-Wirkungskurve in einem Arbeitspunkt bestimmbar ist, ohne die eigentliche Behandlung zu stören und den Patienten zu gefährden.

Die Erfindung löst diese Aufgabe bei einem eingangs genannten Verfahren mit den kennzeichnenden Merkmalen des Patentanspruchs 1. Weiters löst die Erfindung die Aufgabe bei einer eingangs genannten Kombination eines Arzneimittelblisters und eines Speichers mit den kennzeichnenden Merkmalen des Patentanspruchs 10. Ferner löst die Erfindung diese Aufgabe bei einer Vorrichtung zur Herstellung von Arzneimitteln der eingangs genannten Art mit den kennzeichnenden Merkmalen des Patentanspruchs 15. Schließlich löst die Erfindung diese Aufgabe bei einer Vorrichtung zur Herstellung von Arzneimitteln der eingangs genannten Art mit den kennzeichnenden Merkmalen des Patentanspruchs 21.

Erfindungsgemäß ist bei einem Verfahren zur Erstellung einer Anzahl von Arzneimitteln mit einem vorgegebenen Wirkstoff sowie zur gleichzeitigen Bereitstellung eines die jeweilige im Arzneimittel enthaltene Wirkstoffmenge angebenden Wirkstoffwerts vorgesehen, dass für die jeweils festzulegende Wirkstoffmenge der Arzneimittel ein Normwert und eine maximale Abweichung von diesem Normwert vorgegeben werden, wobei ein Wirkstoffwert innerhalb des Intervalls c_{N} +/- Δc gewählt oder vorgegeben wird und für die Erstellung des jeweiligen Arzneimittels eine Wirkstoffmenge herangezogen wird, die dem Wirkstoffwert entspricht, und dass der erstellte Wirkstoffwert dem Arzneimittel zugeordnet und nach der Erstellung des Arzneimittels gemeinsam mit diesem zur Verfügung gehalten wird.
Um eine Herstellung der Arzneimittel und eine anschließende Verpackung der Arzneimittel in Arzneimittelblistern zu ermöglichen, ist vorgesehen, dass das erstellte Arzneimittel nach seiner Herstellung in eine vorgegebene Tasche eines Arzneimittelblisters, insbesondere eines Arzneimittelblisters, eingelegt wird, dass der dem Arzneimittel zugeordnete Wirkstoffwert sowie ein die vorgegebene jeweilige Tasche des Arzneimittelblisters charakterisierender Schlüssel gemeinsam an einen, vorzugsweise auf einem auf dem Arzneimittelblister befindlichen Controller befindlichen, Speicher übertragen werden, und dass ein die jeweiligen Tasche charakterisierender Schlüssel sowie die jeweilige Wirkstoffmenge des in der jeweiligen Tasche befindlichen Arzneimittels, insbesondere vom Controller, zum Abruf zur Verfügung gehalten werden. Zur Überwachung der Einnahme kann vorgesehen sein, dass die die Arzneimittel enthaltenden Taschen des Arzneimittelblisters nach dem Einlegen der Arzneimittel verschlossen werden, und vor der Einnahme des Arzneimittels die jeweilige Tasche vom Patienten geöffnet wird, dass von dem auf dem Arzneimittelblister befindlichen Controller das Öffnen der jeweiligen Tasche detektiert wird, dass der jeweilige Wirkstoffwert des in der zuletzt geöffneten Tasche befindlichen Arzneimittels separat und/oder zusätzlich, insbesondere vom Controller, zur Verfügung gehalten wird, und dass gegebenenfalls der jeweilige Wirkstoffwert an einen Dokumentationsserver übertragen wird, wobei der jeweilige Wirkstoffwert gemeinsam mit dem jeweiligen Ausgabezeitpunkt in einem dem jeweiligen Patienten zugeordneten Dokumentationsspeicher des Dokumentationsservers abgespeichert wird.

Mit einem erfindungsgemäß erzielten Arzneimittel bzw. Medikament kann die jeweilige Dosis-Wirkungskurve ermittelt werden. Nach der Verabreichung der so erstellten Medikamente ermittelten physiologischen Messgrößen können die zu den mit dem erfindungsgemäßen Verfahren erstellten Wirkstoffwerten in Relation gesetzt werden, wodurch auf einfache Weise eine Stellgrößen-Messgrößen-Kurve, insbesondere eine Dosis-Wirkungskurve, ermittelt werden kann, ohne dass sich die grundsätzliche Einstellung der Dosierung bzw. der Arbeitspunkt der Dosierung ändert.

Vorteilhaft kann zur Erzielung aussagekräftiger Dosis-Wirkungskurven auf Grundlage der erstellten Arzneimittel vorgesehen sein, dass die Wirkstoffwerte der einzelnen Arzneimittel als, insbesondere einer vorgegebenen Verteilung folgend, insbesondere gleichverteilt oder diskret verteilt, Zufallswerte innerhalb des vorgegebenen Intervalls c_{N} +/- Δc vorgegeben werden.

Zur präzisen Bestimmung der Steigung der Stellgrößen-Messgrößen-Kurve im Arbeitspunkt kann vorgesehen sein, dass die maximale Abweichung vom Normwert mehr als 5%, insbesondere mehr als 10%, des Normwerts beträgt.

Eine besonders genaue Dosierung und Handhabung kann mit einem in flüssiger Form vorliegenden Arzneimittel erzielt werden. Hierbei ist vorgesehen, dass eine den Wirkstoff enthaltende Wirkstoffflüssigkeit, insbesondere eine Lösung, Emulsion oder Suspension, sowie eine Anzahl von Arzneimittelträgern, insbesondere Pulvertabletten, zur Verfügung gestellt werden, dass eine vorgegebene Menge der Wirkstoffflüssigkeit auf den Arzneimittelträger aufgetropft wird und vom Arzneimittelträgerstoff des Arzneimittelträgers aufgenommen wird, wobei während des Falls des jeweiligen Tropfens in Richtung des Arzneimittelträgers das Volumen des jeweiligen Tropfens bestimmt wird, und dass für das jeweilige Arzneimittel die jeweilige Größe des Tropfens als Wirkstoffwert dem erstellten Arzneimittel zugeordnet und das Arzneimittel gemeinsam mit dem ihm zugeordneten Wirkstoffwert zur Verfügung gehalten wird.

Um eine Personalisierung der auszugebenden oder ausgegebenen Arzneimitteln und eine unmittelbare Aussage über einen Patienten zu ermöglichen, kann vorgesehen sein, dass die erstellten Arzneimittel einem Patienten zugeordnet, vorzugsweise an den Patienten ausgegeben, werden und der jeweilige Wirkstoffwert gemeinsam mit dem jeweiligen Ausgabezeitpunkt in einem dem jeweiligen Patienten zugeordneten Dokumentationsspeicher abgespeichert werden.
Hierbei ist zudem von Vorteil, dass Arzneimittel unmittelbar hergestellt und an einen Patienten ausgegeben werden können.

Alternativ kann, um eine Herstellung der Arzneimittel und eine anschließende Verpackung der Arzneimittel in Arzneimittelblistern zu ermöglichen, auch vorgesehen sein, dass das erstellte Arzneimittel in eine vorgegebene Tasche eines Arzneimittelblisters eingelegt wird, der einen Controller aufweist, auf dem eine vorgegebene, den Arzneimittelblister kennzeichnende Kennung (m₁, ..., mₙ) abgespeichert ist, dass die dem Arzneimittel zugeordnete Wirkstoffmenge (c₁, ..., cₙ), die dem jeweiligen Arzneimittelblister zugeordnete Kennung (m₁, ..., mₙ) sowie ein die vorgegebene jeweilige Tasche des Arzneimittelblisters charakterisierender Schlüssel (k₁, ..., kₙ) gemeinsam an einen Server übertragen werden, und dass der jeweilige Wirkstoffwert (c₁, ..., cₙ) des in der jeweiligen Tasche befindlichen Arzneimittels bei Übermittlung der den Arzneimittelblister charakterisierenden Kennung (m₁, ..., mₙ) sowie des die jeweiligen Tasche charakterisierenden Schlüssels (k₁, ..., kₙ) vom Server zum Abruf zur Verfügung gehalten wird.

In diesem Fall kann zur Überwachung der Einnahme vorgesehen sein, dass die die Arzneimittel enthaltenden Taschen des Arzneimittelblisters nach dem Einlegen der Arzneimittel verschlossen werden, und vor der Einnahme des Arzneimittels die jeweilige Tasche vom Patienten geöffnet wird, dass von dem auf dem Arzneimittelblister befindlichen Controller die jeweils geöffnete Tasche detektiert wird, dass der jeweilige Schlüssel (k₁, ..., kₙ) der zuletzt geöffneten Tasche gemeinsam mit der Kennung (m) des jeweiligen Arzneimittelblisters an den Server übertragen wird und der Server die auf ihm gespeicherte und dem Schlüssel sowie der Kennung (m) zugeordnete Wirkstoffmenge (c₁, ..., cₙ) ermittelt und zur Verfügung hält, und dass gegebenenfalls der jeweilige Wirkstoffwert an eine Stellgrößen-Speichereinheit übertragen wird, wobei der jeweilige Wirkstoffwert gemeinsam mit dem jeweiligen Ausgabezeitpunkt in einem dem jeweiligen Patienten zugeordneten Speicher der Stellgrößen-Speichereinheit abgespeichert werden.

Weiters betrifft die Erfindung eine Kombination umfassend zumindest einen Arzneimittelblister umfassend eine Anzahl von Arzneimitteln, insbesondere Tabletten oder Kapseln, die in im Arzneimittelblister ausgebildeten, und insbesondere ausschließlich das jeweilige Arzneimittel enthaltenden, Taschen angeordnet sind, wobei die einzelnen Arzneimittel jeweils eine unterschiedliche Wirkstoffmenge (c₁, ..., cₙ) aufweisen. Diese Kombination umfasst einen Speicher, der jeweils für jede der Taschen des Arzneimittelblisters jeweils einen Speicherplatz aufweist, in dem ein Wirkstoffwert (c₁, ..., cₙ) abgespeichert ist, der der Wirkstoffmenge des in der jeweiligen Tasche befindlichen Arzneimittels entspricht, wobei der Speicher auf Anfrage bei Übermittlung einer die Tasche eindeutig kennzeichnenden Kennung (m₁, ..., mₙ) den jeweiligen dem Arzneimittel in der jeweiligen Taschen zugeordneten Wirkstoffwert (c) zur Verfügung hält.

Eine solche Kombination eines Arzneimittelblisters und eines Speichers kann vorteilhaft für die Ermittlung von Dosis-Wirkungskurven verwendet werden. Insbesondere steht für jedes eingenommene oder einzunehmende Medikament die jeweilige Medikamentendosis als Wirkstoffwert zur Verfügung. Um den Zusammenhang zwischen Dosierung und Wirkung des Arzneimittels zu messen, kann das jeweilige Arzneimittel an den Patienten verabreicht werden und anschließend können die Auswirkungen ermittelt und quantifiziert werden, z.B. durch Messung des Blutdrucks bei einem blutdrucksenkenden Medikament. Da für das jeweilige Medikament die Medikamentendosis einfach über den Speicher abrufbar zur Verfügung steht, kann die Dosis-Wirkungskurve rasch erstellt und für neue Therapieempfehlungen zur Verfügung gehalten werden.

Um zu erkennen, welches von mehreren Arzneimittel aus einem Behälter entnommen wurde und um den für die Dosis-Wirkungskurve zu verwendenden Wirkstoffwert zu erhalten, kann vorgesehen sein, dass auf dem Arzneimittelblister ein Controller vorgesehen ist,
a) wobei auf dem Controller eine den Arzneimittelblister eindeutig kennzeichnende Kennung (m₁, ..., mₙ) abgespeichert ist und/oder
b) wobei der Controller das Öffnen von auf dem Arzneimittelblister ausgebildeten Tasche überwacht und eine die jeweils zuletzt geöffnete Tasche eindeutig kennzeichnende Kennung (m₁, ..., mₙ) zur Verfügung hält.

Eine einfache Möglichkeit des Zugriffs auf die einzelnen Wirkstoffwerte sieht vor, dass der Speicher in dem auf dem Arzneimittelblister angeordneten Controller integriert ist, wobei im Speicher für jedes in den einzelnen Taschen befindliche Arzneimittel jeweils ein Speicherbereich vorgesehen ist, in dem die jeweilige Wirkstoffmenge (c₁, ..., cₙ) des Arzneimittels abgespeichert ist, und dass der Controller auf Anfrage bei Angabe eines die jeweiligen Tasche charakterisierenden Schlüssels (k₁, ..., kₙ) die jeweilige Wirkstoffmenge (c₁, ..., cₙ) des in der jeweiligen Tasche befindlichen Arzneimittels angibt.

Alternativ kann zum selben Zweck vorgesehen sein, dass der auf dem Arzneimittelblister ausgebildete Controller einen Identifikationsspeicher aufweist, auf dem eine dem Arzneimittelblister eindeutig kennzeichnende Kennung (m₁, ..., mₙ) vorgesehen ist, dass der Speicher auf einem Server ausgebildet ist, wobei der Server gegebenenfalls eine Anzahl von Speichern aufweist, wobei jeder Speicher jeweils einem Arzneimittelblister zugeordnet ist, wobei im jeweiligen für den Arzneimittelblister vorgesehenen Speicher für jedes in den einzelnen Taschen des Arzneimittelblisters befindliche Arzneimittel jeweils ein Speicherbereich vorgesehen ist, in dem der jeweilige Wirkstoffwert (c₁, ..., cₙ) des in der Tasche des Arzneimittelblisters befindlichen Arzneimittels abgespeichert ist, und dass der Server auf Anfrage bei Angabe der den Arzneimittelblister charakterisierenden Kennung (m₁, ..., mₙ) sowie eines die jeweilige Tasche des Arzneimittelblisters charakterisierenden Schlüssels (k₁, ..., kₙ) die jeweilige Wirkstoffmenge (c₁, ..., cₙ) des in der jeweiligen Tasche befindlichen Arzneimittels übermittelt.
Dies ermöglicht darüber hinaus auch, dass das Öffnen der einzelnen Arzneimittelblister zentral überwacht werden kann, sowie eine einfache zentrale Datenspeicherung sowie eine damit einhergehende erhöhte Fälschungssicherheit.

Vorteilhaft kann zur Erzielung aussagekräftiger Dosis-Wirkungskurven auf Grundlage der erstellten Arzneimittel vorgesehen sein, dass die Wirkstoffwerte (c) der einzelnen Arzneimittel innerhalb des Intervalls c_{N} +/- Δc einer vorgegebenen Verteilung folgen, beispielsweise gleichverteilt oder diskret verteilt sind.

Zur präzisen Bestimmung der Steigung der Stellgrößen-Messgrößen-Kurve im Arbeitspunkt kann vorgesehen sein, dass die maximale Abweichung (Δc) vom Normwert (c_{N}) mehr als 5%, insbesondere mehr als 10%, des Normwerts (c_{N}) beträgt.

Schließlich betrifft die Erfindung auch eine Vorrichtung zur Herstellung von Arzneimitteln. Erfindungsgemäß ist bei einer Vorrichtung zur Herstellung von Arzneimitteln umfassend
a) eine Bereitstellungseinheit zur Bereitstellung von Arzneimittelträgern, insbesondere Pulvertabletten,
b) eine Portionierungseinheit zur Portionierung des Wirkstoffs, insbesondere einer Wirkstoffflüssigkeit, sowie zum Aufbringen des Wirkstoffs, insbesondere zum Auftropfen der Wirkstoffflüssigkeit, auf die von der Bereitstellungseinheit bereit gestellten Arzneimittelträger vorgesehen: c) eine Steuereinheit zur Steuerung der Größe der von der Portionierungseinheit abgegebenen Menge des Wirkstoffs, insbesondere der Tropfen, wobei die Steuereinheit für die jeweils festzulegende Wirkstoffmenge der Arzneimittel einen Normwert (c_{N}) festlegt, und die Steuereinheit der Portionierungseinheit jeweils eine aufzubringende Wirkstoffmenge innerhalb eines Intervalls rund um den Normwert (c_{N}) vorgibt, wobei die Steuereinheit die vorgegebene oder die im Arzneimittel enthaltene Wirkstoffmenge als Wirkstoffwert (c) des erstellten Arzneimittels nach dessen Erstellung zur Verfügung hält und in einem dafür vorgesehenen Speicher dauerhaft abspeichert.

Mit einer solchen Vorrichtung können einfach und rasch Arzneimittel hergestellt werden, deren jeweiliger Wirkstoffwert bekannt ist und für die Erstellung einer Dosis-Wirkungskurve herangezogen werden kann.

Um Medikamente mit einer vorgegebenen und insbesondere einstellbaren Abweichung der Dosis sowie des Wirkstoffwerts zu erzielen, kann vorgesehen sein, dass die Steuereinheit zur Steuerung der Größe der von der Portionierungseinheit abgegebenen Menge des Wirkstoffs ausgebildet ist, dass die Steuereinheit für die jeweils festzulegende Wirkstoffmenge der Arzneimittel einen Normwert (c_{N}) und eine maximale Abweichung (Δc) von diesem Normwert, festlegt, dass die Steuereinheit der Portionierungseinheit jeweils eine aufzubringende Wirkstoffmenge, insbesondere einen Zufallswert (c_{R}) innerhalb des Intervalls c_{N} +/- Δc, als Wirkstoffwert (c) vorgibt, wobei die Steuereinheit den vorgegebenen Wirkstoffwert (c) nach der Erstellung des Arzneimittels zur Verfügung hält.

Um die natürliche Herstellungstoleranz einer bestehenden Portionierungseinheit zu nutzen, kann vorgesehen sein, dass die Portionierungseinheit zur Portionierung der Wirkstoffflüssigkeit sowie zum Auftropfen der Wirkstoffflüssigkeit auf die von der Bereitstellungseinheit bereit gestellten Arzneimittelträgern, ausgebildet ist, und die Positionierungseinheit die Tropfen der Wirkstoffflüssigkeit mit einer vorgegebenen durchschnittlichen Tropfengröße (c_{N}) und einer bekannten maximalen Abweichung (Δc) von der vorgegebenen durchschnittlichen Tropfengröße (c_{N}) abgibt, und dass die Steuereinheit eine Einheit zur Bestimmung der Tropfengrößen von von der Portionierungseinheit auf die Arzneimittelträger aufgebrachten Tropfen vorgesehen ist, welche die Größe der jeweiligen auf die Arzneimittelträger aufgebrachten Tropfen als Wirkstoffwert (c) zur Verfügung hält.

Einige Ausführungsbeispiele der Erfindung werden anhand der folgenden Zeichnungen näher dargestellt.

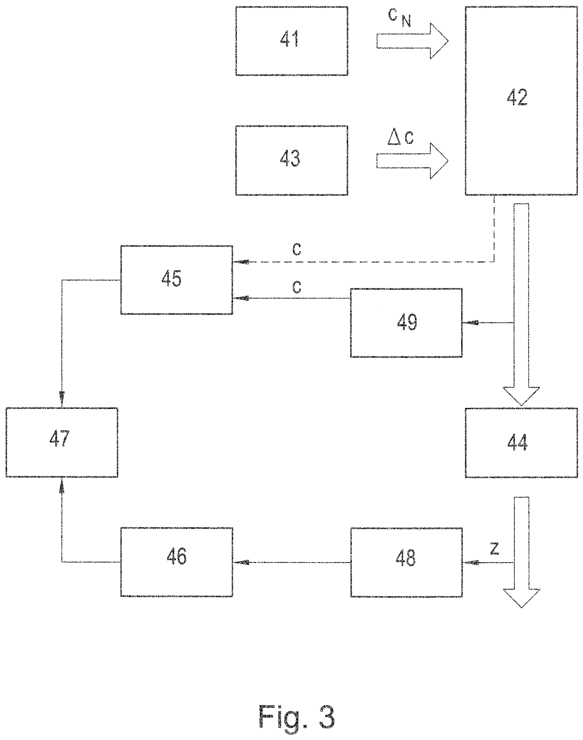

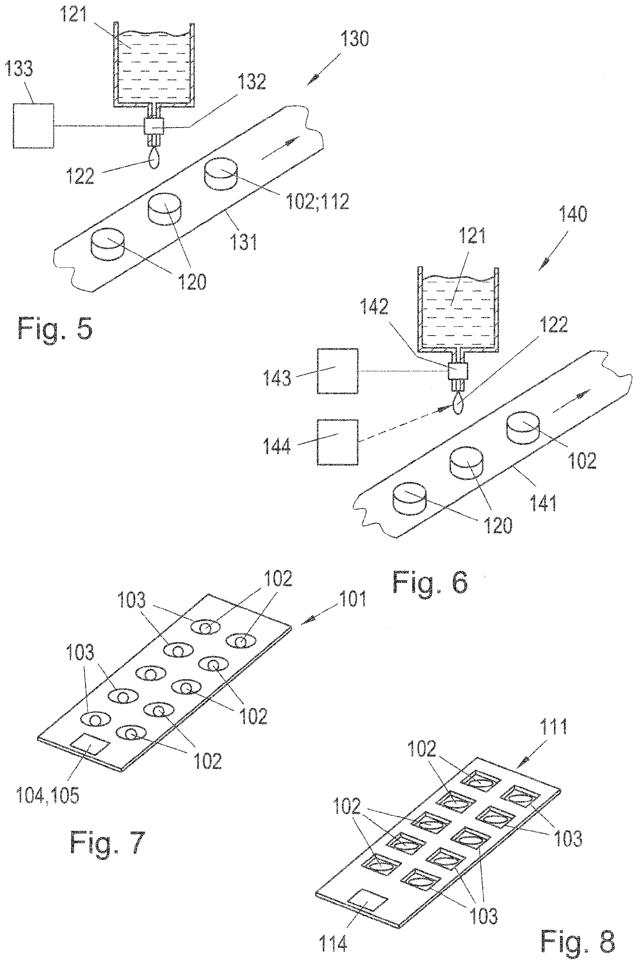

In **Fig. 3** ist schematisch der Ablauf einer Behandlung dargestellt. **Fig. 4** zeigt die bei dem in **Fig. 3** dargestellten Verfahren ermittelte Dosis-Wirkungskurve. **Fig. 5 und 6** zeigen unterschiedliche Ausführungsformen zur Herstellung von Arzneimitteln. **Fig. 7 und 8** zeigen zwei unterschiedliche mit einem erfindungsgemäßen Verfahren hergestellte Arzneimittelblister.

In **Fig. 3** sind der Ablauf einer Behandlung sowie die Auswertung der einzelnen Messgrößen schematisiert dargestellt, die den Ausgangspunkt der Erfindung bildet. Ein Arzt 41 bestimmt dabei eine Behandlungsintensität, beispielsweise eine Arzneimitteldosierung, eine Strahlendosis, Trainingsdauer für Übungen usw. Durch diese Behandlungsintensität wird ein initialer Stellwert vorgegeben, mit dem ein bestimmter physiologischer Zielwert erreicht werden soll. So kann der Arzt 41 beispielsweise mit der Einstellung einer Dosierung eines Medikaments eine Verringerung des systolischen Blutdruckwerts auf einen bestimmten Normwert erzielen wollen. Da jedoch unterschiedliche Patienten 44 unterschiedlich auf die jeweiligen verabreichten Medikamente reagieren, kann die Wirkung des Medikaments stärker oder schwächer als vom Arzt 41 erwartet ausfallen, sodass nach einer bestimmten Zeit eine Korrektur der Behandlungsintensität erforderlich wird. So kann der Arzt 41 etwa die tägliche Dosierung senken, um im Falle einer zu starken Reaktion des Patienten 44 auf das Arzneimittel eine zu niedrige Einstellung des Blutdrucks zu vermeiden.

Wie bereits erwähnt, legt der Arzt 41 zu einem initialen Zeitpunkt sowohl die Stellgröße bzw. die Behandlungsintensität als auch die gewünschte Messgröße z fest. Der Arzt legt neben der Stellgröße c_{N} und Messgröße z auch noch eine Variation Δc der Stellgröße c fest. Der Therapie-Verabreichungseinheit 42 sind sowohl die vom Arzt vorgegebene Stellgröße c_{N} als Arbeitspunkt-Einstellung sowie auch die Variationseinstellung Δc, die von einer Variationseinheit 43 übermittelt wird, zugeführt. Aus diesen Größen c_{N}, Δc bestimmt die Therapie-Verabreichungseinheit 42 eine Stellgröße c bzw. Behandlungsintensität c innerhalb des Intervalls c_{N} +/- Δc, Dem Patienten 44 wird im Rahmen der jeweiligen Therapie-Verabreichungseinheit 42 eine Therapie mit dieser Behandlungsintensität c verabreicht.

Der tatsächliche Wert der Stellgröße c, das heißt inklusive der jeweils aufgeprägten Variation, sowie der Zeitpunkt der Verabreichung wird in einer Stellgrößen-Speichereinheit 45 gespeichert. Die Information kann entweder indirekt aus der Einstellung der von der Variationseinheit 43 beeinflussten Therapie-Verabreichungseinheit 42 oder direkt durch Messung des tatsächlichen Wertes der Stellgröße über eine Stellgrößen-Messeinheit 49 bestimmt werden.

Anschließend wird die Messgröße z, beispielsweise der Blutdruck, über eine Messgrößen-Messeinheit 48 gemessen und der Wert und der Messzeitpunkt in einer Messgrößen-Speichereinheit 46 gespeichert. Aus den Werten für Stellgröße c und Messgröße z und den dazugehörigen Zeitpunkten werden in einer Auswerteinheit 47 der aktuelle Arbeitspunkt und die Empfindlichkeit der therapeutischen Messgröße z einer Person in Bezug auf Veränderung der therapeutischen Stellgröße c berechnet.

Als Maß für diese Empfindlichkeit kann eine Sensitivität F des jeweiligen Patienten auf die jeweilige Therapie bzw. Behandlung in einem Arbeitspunkt ermittelt werden, die im Bereich des Normwerts c_{N} oder des Arbeitspunkts gleich der relativen Zunahme dz/dc der physiologische Messgrößen z bei Erhöhung des jeweiligen Wirkstoffwerts c ist.

Eine präzisere Einstellung der Messgröße z kann - sofern die jeweilige Abhängigkeit der Messgröße z von der Stellgröße im jeweiligen Arbeitspunkt bekannt ist - einfacher erfolgen. Zudem besteht die Möglichkeit zu erfahren, wie stark sich Änderungen der Stellgröße c auf die Messgröße z auswirken. Es ist somit bei Kenntnis der Abhängigkeit der Stellgröße c von der Messgröße z in einem Arbeitspunkt, nicht nur möglich, lineare Verschiebungen der Stellgrößen-Messgrößen-Kurve zu quantifizieren, sondern auch Änderungen ihrer Steigung im Arbeitspunkt zu gewinnen - und damit eine Aussage darüber zu treffen, wie empfindlich die Messgröße z auf geringfügige Änderungen der Stellgröße c aus dem Arbeitspunkt heraus reagieren wird.

Um eine jederzeit aktuelle Stellgrößen-Messgrößen-Kurve zu erhalten, wird bei der Therapie, beispielsweise bei jeder Medikamenteneinnahme oder Behandlung, die Stellgröße c bzw. Behandlungsintensität, beispielsweise die Dosierung des Medikaments, um einen Arbeitspunkt herum verändert.

Der jeweilige Wert der Stellgröße c wird der jeweiligen Behandlung zugeordnet, beispielsweise kann bereits bei der Produktion eines Arzneimittelblisters die Wirkstoffmenge der im Arzneimittelblister befindlichen Medikamente oder Arzneimittel gespeichert werden (Fig. 5, 6). Alternativ kann auch bei der Verabreichung der Arzneimittel die jeweilige Wirkstoffmenge gemessen und gespeichert werden (Fig. 7, 8). Die Abspeicherung der Behandlungsintensität erfolgt unabhängig vom Wert der gemessenen physiologischen Messgröße z, beispielsweise vom gemessenen Blutdruck. Dadurch entsteht in der Stellgrößen-Messgrößen-Kurve eine Punktmenge, die sowohl die Variation der Stellgröße als auch die Variation der Messgröße z wiederspiegelt. Aus dieser in **Fig. 4** dargestellten Punktmenge kann durch mathematische Verfahren, beispielsweise Fitting einer Geraden, die aktuelle Steigung der Kurve im Arbeitspunkt, d.h. für die aktuelle Dosierung, ermittelt werden, welcher die aktuelle Empfindlichkeit der Messgröße z auf Veränderungen der Stellgröße im Arbeitspunkt entspricht.

Die Stellgröße bzw. Behandlungsintensität wird während der Therapie nicht völlig konstant gehalten, sondern bei jeder Therapieanwendung, z.B. bei jeder Medikamenteneinnahme, verändert. Der jeweils aktuell verabreichte Wert der Stellgröße wird gespeichert, gleich wie der Wert der aktuellen Messgröße z. Dadurch entsteht in der Stellgrößen-Messgrößen-Kurve eine zweidimensionale Punktmenge 31, die sowohl die Variation der Stellgröße als auch die Variation der Messgröße z wiederspiegelt. Die Messgröße z ist jetzt - wie oben beschrieben - immer noch von Störgrößen, Messfehlern und Änderungen der ursprünglichen Stellgrößen-Messgrößen-Kurve 32 beeinflusst, zusätzlich aber auch noch vom jeweiligen Wert der Stellgröße. Unter der Annahme, dass Störgrößen und Messfehler stochastisch verteilt sind und die Stellgrößen-Messgrößen-Kurve sich nur langsam ändert, kann daher aus der vorliegenden Punktmenge nicht nur der mittlere Wert der Messgröße z, sondern auch, z.B. durch Fitting einer Geraden 33, die Steigung dz/dc der Kurve im Arbeitspunkt und damit die Empfindlichkeit der Messgröße z auf Veränderungen der Stellgröße bestimmt werden. Gegebenenfalls kann auch eine neuerliche Approximation einer bekannten Kurve 34 vorgenommen werden. Je mehr Messpunkte zur Verfügung stehen, desto geringer kann dabei der Einfluss der Variation der Stellgröße im Vergleich zum Einfluss von Messfehler und Störgrößen sein und bei entsprechend langer Beobachtung können auch minimale Variationen der Stellgröße ausreichen.

Wie bereits erwähnt, ist die Stellgröße c der Behandlung nicht zwingend die Dosierung eines Medikaments. Es besteht auch die Möglichkeit, andere Behandlungsintensitäten therapeutischer Interventionen als Stellgrößen heranzuziehen, die eine kontrollierbare bzw. variierte Stellgröße haben. Demnach kann bei Lichttherapie die Intensität des Lichts geändert werden, bei elektromagnetischer Strahlung auch die Intensität oder die Wellenlänge, bei Wärme-/Kältetherapie die Temperatur, bei all diesen Therapien die Dauer der Einwirkung, etc. Ähnliche einstellbare Stellgrößen können in der Akustik, Mechanik, Ernährung, etc. gefunden werden.

Auch die Messgröße z kann im Prinzip jede quantitativ messbare Größe sein, die durch die Therapie beeinflusst wird, also z.B. Blut- oder Harnwerte, Blutdruck, EKG-Parameter, Pulsfrequenz, Körpertemperatur, etc.

Während der Therapie fallen regelmäßig Paare (c, z) aus Stellgrößen- und Messgrößen-Werten an. Für das Fitting der Stellgrößen-Messgrößen-Kurve können sämtliche dieser bisher gemessenen Stellgrößen-Messgrößen-Paare verwendet werden. Um Veränderungen zu erkennen, kann es aber auch sinnvoll sein, nur die Daten aus einem vorgegebenen Zeitraum, beispielsweise aus der letzten Woche oder des letzten Monats, zu verwenden. Falls bekannt ist, dass gewisse Parameter als Störgrößen ebenfalls die Messgröße z beeinflussen - so hängt der Blutdruck stark von der Tageszeit ab und ist morgens oft anders als abends - können nur bestimmte Wertepaare ein- oder ausgeschlossen werden - abhängig vom Wert der Störgröße. Ebenfalls ausgeschlossen werden können Wertepaare, die offensichtlich statistische Ausreißer sind, weil z.B. die Messgröße z im Sinne eines Ausreißers signifikant verschieden zu den anderen bisher gemessenen Messgrößen z ist.

Die Variation der Stellgröße kann einer bestimmten Vorgabe folgen, also aus einer vorgegebenen Folge von Werten im Sinne eines deterministischen Signals bestehen, oder auch rein zufällig sein, also die Charakteristik eines Rauschens haben. Die Erfindung sieht vor, dass jede Veränderung der Stellgröße c gespeichert und zur Verfügung gehalten wird. So können etwa variierende Behandlungsintensitäten entweder bei der Produktion von Arzneimitteln 102 oder bei deren Verabreichung der Arzneimittel 102 gespeichert oder erst im Rahmen der Behandlung ermittelt werden, um anschließend die Stellgrößen-Messgrößen-Punktwolken bestimmen zu können.

Die aktuellen Werte der Stellgröße c werden für jede Therapie separat abgespeichert und stehen für die spätere Verarbeitung zur Verfügung. So kann etwa die genaue, jedoch prozessbedingt um einen statistischen Mittelwert schwankende Menge eines auf Trägertabletten bzw. Arzneimittelträger 120 aufgetropften Wirkstoffes im Zuge der Erzeugung und Verpackung der Tabletten in einer Speichereinheit bzw. einem Stellgrößen-Speichereinheit 45 einer Medikamentenausgabevorrichtung, beispielsweise eines Arzneimittelblisters 101, 111, mitgespeichert werden.

Neben dem Wert der Stellgröße c beeinflussen auch der Zeitpunkt, zu dem die Verabreichung durchgeführt wird, also z.B. wann ein Medikament bzw. Arzneimittel 102; genommen wird, und der Zeitpunkt, wann die Messung der Messgröße z durchgeführt wird, d.h. entweder sofort nach Einnahme, nach Stunden, oder erst nach Tagen, etc., das Messergebnis. Um auch diese Effekte mit zu berücksichtigen kann nicht nur der Wert der Stellgröße c variiert werden, sondern auch der Zeitpunkt jeder einzelnen Therapieanwendung und/oder der Zeitpunkt der Messung der Messgröße z.

Zusätzliche Aussagekraft kann der Untersuchung verliehen werden, indem nach dem Setzen einer therapeutischen Intervention nicht nur einmalig - zu einem einzigen Zeitpunkt - eine Messung erfolgt, sondern über einen längeren Zeitraum und mehrfach, gegebenenfalls, kontinuierlich der Verlauf einer Messgröße z beobachtet wird. Wenn diese Messserie bei jeder Anwendung durchgeführt wird, kann aus dem Vergleich der Signalverläufe nach den einzelnen Interventionen eine Aussage über den Einfluss der Stellgröße c auf die Messgröße z bestimmt werden.

Weiters kann durch Verwenden einer Kreuzkorrelationsanalyse festgestellt werden, ob Zeitkonstanten der Beeinflussung der Messgröße z vom Wert der Stellgröße abhängig sind bzw. generell eine eventuell gegebene zeitliche Verschiebung zwischen der Applikation der Stellgröße c und beobachtbaren Veränderungen der Messgröße z berücksichtigt werden.

Generell kann im Zuge der Auswertung ein Modell für den Zusammenhang der Stell- und der Messgröße c; z im Sinne eines Übertragungssystems formuliert und mittels mathematischer Methoden die Strukturparameter dieses Modells im Sinne einer Systemanalyse geschätzt werden.

In Fig. 5 ist ein Verfahren gemäß einer Ausführungsform der Erfindung näher dargestellt, das zur Erstellung einer Anzahl von Arzneimitteln 102 dient, die mit einem vorgegebenen Wirkstoff ausgestattet sind. Ziel des Verfahrens ist es, gleichzeitig mit der Herstellung der Arzneimittel 102 die jeweilige im Arzneimittel 102 enthaltene Wirkstoffmenge in Form eines Wirkstoffwerts c zur Verfügung zu halten. Insbesondere soll der Wirkstoffwert c in einer Stellgrößen-Speichereinheit 45 zum Abruf zur Verfügung gehalten werden.

Am Beginn des Verfahrens wird die durchschnittliche zu verabreichende Soll-Wirkstoffmenge der Arzneimittel 102 in Form eines Normwerts c_{N} durch einen Arzt vorgegeben oder auf einen Standardwert festgelegt. Weiters wird eine maximale Abweichung Δc von diesem Normwert c_{N} vorgegeben. Der Wirkstoffwert c liegt somit innerhalb eines Intervalls c_{N} ± Δc. Der Wirkstoffwert c kann dabei entweder gewählt werden oder die Beschickung des Arzneimittels 102 mit dem vorgegebenen Wirkstoff wird mit einer bestimmten Fehlertoleranz vorgenommen, sodass die letztlich erstellten Arzneimittel 102 eine Wirkstoffmenge enthalten, der ein im Intervall c_{N} ± Δc liegender Wirkstoffwert entspricht.

Sofern der Wirkstoffwert c vorgegeben wird, kann der Wirkstoffwert c dem jeweiligen Arzneimittel zugeordnet und im zuvor genannten Speicher 105 abgespeichert werden.

Unterliegt der erstellte Wirkstoffwert c dadurch einer gewissen Variation, dass die Herstellung des jeweiligen Arzneimittels 102 nicht exakt durchgeführt werden kann, wird die Menge an im Arzneimittel 102 befindlichen Wirkstoffe in einem nachfolgenden Schritt gemessen und das Messergebnis als Wirkstoffwert c dem jeweiligen Arzneimittel 102 zugeordnet. Nach der Erstellung des Arzneimittels 102 werden sowohl das Arzneimittel 102 als auch der Wirkstoffwert des Arzneimittels 102 zur Verfügung gehalten.

Abhängig vom jeweiligen Arzneimittel bzw. abhängig von der jeweiligen im Arzneimittel enthaltenen Wirkstoffsubstanz oder Wirksubstanz beträgt die maximale Abweichung Δc vom Normwert c_{N} zwischen 10 % und 20 %. Insbesondere beträgt die Abweichung vom Normwert mehr als 5 % bzw. mehr als 10 %.

Schematische Darstellungen des Vorgehens bei der Herstellung von Arzneimitteln gemäß zweier bevorzugten Ausführungsformen der Erfindung sowie zwei Vorrichtungen 130, 140 zur Erstellung von Arzneimitteln 102 sind in den **Fig. 5 und 6** dargestellt. Eine einen Arzneimittelwirkstoff enthaltende Wirkstoffflüssigkeit 121, beispielsweise eine Emulsion, Lösung oder Suspension, im vorliegenden Fall eine Wirkstofflösung, ist in einem Vorratsbehälter vorhanden. Beide in den **Fig. 5 und 6** dargestellten Vorrichtungen 130, 140 verfügen über jeweils eine Bereitstellungseinheit 131, 141, mit der Arzneimittelträger 120 in Form von Pulvertabletten zur Verfügung gestellt werden. Der mit der Wirkstoffflüssigkeit 121 gefüllte Behälter weist an seinem unteren Ende einen Auslass auf, der in eine Portionierungseinheit 132, 142 mündet. Die Portionierungseinheit 132, 142 stellt Tropfen 122 der Wirkstoffflüssigkeit 121 zur Verfügung und lässt diese auf die von der Bereitstellungseinheit 131, 141 bereitgestellten Arzneimittelträger 120 auftropfen. Weiters verfügt die Vorrichtung über eine Steuereinheit 133, 143, die die Portionierungseinheit 132, 142 ansteuert und die Menge des von der Portionierungseinheit 132, 142 abzugebenden Wirkstoffs festlegt. Die Steuereinheit 133 erhält ihrerseits jeweils die festzulegende Wirkstoffmenge für das Arzneimittel, wobei ein Normwert von c_{N} sowie ein Intervall Δc rund um diesen Normwert c_{N} vorgegeben werden.

Wenn die Portionierungseinheit 132 zur Herstellung der Arzneimittel 102, dargestellt in **Fig. 5**, hinreichend genau arbeitet, kann zur Erstellung von Arzneimitteln 102 mit einer gewissen Varianz Δc der einzelnen Wirkstoffwerte c für jedes einzelne Arzneimittel 102 ein separater Wirkstoffwert c in Form eines Zufallswerts vorgegeben werden. Dieser Zufallswert befindet sich stets innerhalb des Intervalls c_{N} ± Δc. Die Verteilung der einzelnen Zufallswerte folgt einer bestimmten Verteilung. Im vorliegenden Ausführungsbeispiel wurde als Verteilung für die Zufallswerte eine Gleichverteilung innerhalb des vorgegebenen Intervalls c_{N} ± Δc gewählt.

Sofern also die Portionierungseinheit 132 Arzneimittel 102 mit einer hinreichend genau festlegbaren Wirkstoffmenge der Wirkstoffflüssigkeit beaufschlagt, kann der jeweilige im Arzneimittel 102 enthaltene Wirkstoffwert c unmittelbar dem jeweiligen Arzneimittel 102 zugeordnet werden und gemeinsam mit einer Identifikationsnummer für dieses Arzneimittel 102 zur Verfügung gehalten werden. In diesem Fall ist die Steuereinheit 133 zur Steuerung der Größe der von der Portionierungseinheit 132 abgegebenen Menge der Wirkstoffflüssigkeit 121 ausgebildet. Die Steuereinheit 133 legt die jeweilige im Arzneimittel 102 enthaltene Wirkstoffmenge innerhalb eines Intervalls c_{N} ± Δc um den Normwert c_{N} beispielsweise als Zufallswert fest.

Mit einer weniger präzisen Steuereinheit 143 bzw. Portionierungseinheit 142 kann eine statistische Verteilung der auf die einzelnen Arzneimittelträger 120 aufgebrachten Wirkstoffmengen bereits durch die herstellungsbedingten Toleranzen erzielt werden. Eine solche Ausführungsform der Erfindung ist in **Fig. 6** dargestellt. Auch diese Ausführungsform verfügt über eine Bereitstellungseinheit 141 für Arzneimittelträger 120, einen Behälter für die Wirkstoffflüssigkeit 121, eine Steuereinheit 143, eine Portionierungseinheit 142. Zusätzlich weist diese Vorrichtung 140 jedoch eine Einheit 144 zur Bestimmung der Größe von der Portionierungseinheit 142 auf die Arzneimittelträger 120 aufgebrachten Tropfen 122. Um die jeweilige dem Arzneimittel 102 zugeordnete Wirkstoffmenge in Form eines Wirkstoffwerts c zu ermitteln, wird im Gegensatz zu der in **Fig. 5** dargestellten Ausführungsform nicht das der Portionierungseinheit 132, 142 aufgeprägte Steuersignal der Steuereinheit 143 herangezogen, hier würde das aufgeprägte Signal gerade durch die fertigungsbedingten Toleranzen verfälscht. Es wird vielmehr die Größe des von der Portionierungseinheit 142 abgegebenen Tropfens 122 der Wirkstoffflüssigkeit 121 separat und direkt gemessen. Der so ermittelte Messwert für die Größe des auf den Arzneimittelträger 120 aufgetropften Tropfens 122 wird als Wirkstoffwert c für das jeweilige Arzneimittel 102 zur Verfügung gehalten und abgespeichert.

Nach der Herstellung der Arzneimittel bestehen nun grundsätzlich zwei Möglichkeiten der Ausgabe und Verabreichung der erstellten Arzneimittel 102 an einen Patienten: Gemäß einer ersten Ausführungsform der Erfindung werden die einzelnen Arzneimittel unmittelbar nach deren Herstellung unmittelbar an den Patienten ausgegeben. Der Patient erhält die erstellten Arzneimittel, wobei der jeweilige dem Arzneimittel 102 zugeordnete Wirkstoffwert c unmittelbar in einen in **Fig. 3** dargestellten Stellgrößen-Speichereinheit 45 übertragen wird, der dem jeweiligen Patienten zugeordnet ist. Auf diese Art lässt sich beispielsweise in Spitälern oder bei durchgeführten Studien rasch und kostengünstig eine Vielzahl von Arzneimitteln 102 erstellen, ohne dass ein komplizierter Verpackungsprozess erforderlich wäre.

Alternativ kann ein in **Fig. 7** dargestellter Arzneimittelblister 101 erstellt werden, in dem die so erstellten Arzneimittel 102 eingelegt werden. Die einzelnen Arzneimittel 102 werden unmittelbar nach ihrer Erstellung jeweils in einzelne Taschen 103 des Arzneimittelblisters 101 eingelegt. Der dem jeweiligen Arzneimittel 102 zugeordnete Wirkstoffwert c sowie ein die jeweilige vorgegebene Tasche 103 des Arzneimittelblisters 101 charakterisierender Schlüssel k₁, ..., kₙ wird an einen Speicher 105 übertragen und auf diesem abgespeichert. In der in **Fig. 5** dargestellten Ausführungsform der Erfindung befindet sich der Speicher 105 gemeinsam mit einem Controller 104 auf einem Mikrochip, der unmittelbar auf dem Arzneimittelblister 101 angeordnet ist. Der Mikrochip verfügt ferner über eine RFID-Funktionalität und ermöglicht dem Controller 104 die Kommunikation mit externen Datenkommunikationseinheiten und insbesondere die Datenübertragung von Wirkstoffwerten c an die Stellgrößen-Speichereinheit 45. Für jedes der Arzneimittel 102 wird jeweils ein die jeweilige Tasche 103 charakterisierender Schlüssel k₁, ..., kₙ sowie die jeweilige Wirkstoffmenge des in der jeweiligen Tasche 103 befindlichen Arzneimittels 102 als digitaler Wirkstoffwert c abgespeichert. Der Controller 104 hält die jeweilige Wirkstoffmenge sowie den jeweils zugeordneten Schlüssel k₁, ..., kₙ zum gemeinsamen Abruf zur Verfügung.

Nach dem Einlegen der Arzneimittel 102 in die Taschen 103 des Arzneimittelblisters 101 oder -behälters werden die Taschen 103 verschlossen. Die Taschen 103 werden erst vom Patienten 44 unmittelbar vor der Einnahme des jeweiligen Arzneimittels 102 geöffnet. Der Controller 104 detektiert das Öffnen der jeweiligen Tasche 103 und hält den die jeweils zuletzt geöffnete Tasche 103 charakterisierenden Schlüssel k₁, ..., kₙ gemeinsam mit dem Wirkstoffwert c des in dieser Tasche 103 befindlichen Arzneimittels zur Verfügung. Es ist somit möglich, von dem jeweiligen Arzneimittelblister 101 bzw. von dem auf ihm befindlichen Controller 104 abzufragen, wie hoch der Wirkstoffwert c des in der zuletzt geöffneten Tasche 103 befindlichen Arzneimittels 102 ist oder war. Zur Feststellung, wann und ob eine Tasche 103 geöffnet worden ist, steht aus dem Stand der Technik eine Vielzahl von unterschiedlichen Verfahren zur Verfügung. Insbesondere ist es möglich, im Bereich der die Taschen 103 verschließenden Folie Leiterbahnen anzubringen, die beim Öffnen der Taschen 103 unterbrochen werden. Auf diese Weise kann der Controller 104 feststellen, dass die jeweilige Tasche 103 geöffnet wird und den der jeweiligen Tasche 103 zugeordneten Wirkstoffwert c auf Anfrage zur Verfügung stellt.

Bei der Ermittlung der Dosis-Wirkungskurve ist es von Vorteil, dass der jeweilige Wirkstoffwert c der zuletzt geöffneten Tasche 103 an die Stellgrößen-Speichereinheit 45 übertragen wird, wobei der jeweilige Wirkstoffwert c gemeinsam mit dem jeweiligen Ausgabezeitpunkt, das heißt dem Zeitpunkt, zu dem der Patient die Tasche 103 geöffnet hat, abgespeichert wird. Dem so abgespeicherten Datensatz wird zudem eine persönliche Identifikationsnummer des jeweiligen Patienten 44 hinzugefügt.

Gemäß einer weiteren alternativen Ausführungsform der Erfindung, die im übrigen der in **Fig. 7** dargestellten Ausführungsform der Erfindung entspricht, kann auch vorgesehen sein, die einzelnen Wirkstoffwerte nicht auf einem auf dem Arzneimittelblister 111 befindlichen Speicher abzulegen, sondern auf einem auf einem - nicht dargestellten - zentralen Server befindlichen Speicher zur Verfügung zu halten. Eine solche Ausführungsform der Erfindung ist in **Fig. 8** näher dargestellt. Wiederum befinden sich die einzelnen Arzneimittel in einer vorgegebenen Tasche 103 eines Arzneimittelblisters bzw. - Arzneimittelblisters 111. Der Arzneimittelblister 111 weist einen Controller 114 auf, auf dem eine vorgegebene, den Arzneimittelblister 111 kennzeichnende Kennung m₁, ..., mₙ abgespeichert ist. Darüber hinaus ist, wie auch im vorigen Ausführungsbeispiel der Erfindung, jeder einzelnen Tasche 103 des Arzneimittelblisters 111 jeweils ein charakterisierender Schlüssel k₁, ..., kₙ zugeordnet. Bei der Erstellung eines Arzneimittels 102 wird jeweils ein Datensatz erstellt, der die dem Arzneimittel 102 zugeordnete Wirkstoffmenge c, die den Arzneimittelblister 111 zugeordnete Kennung sowie ein die jeweilige Tasche 103 des Arzneimittelblisters 111 charakterisierender Schlüssel k₁, ..., kₙ aufweist. Diese Datensätze werden gemeinsam an den zentralen Server übertragen, der den jeweiligen Wirkstoffwert c des in der Tasche 103 befindlichen Arzneimittels 102 bei Übermittlung der den Arzneimittelblister 111 charakterisierenden Kennung m₁, ..., mₙ sowie des die jeweilige Tasche 103 charakterisierenden Schlüssels k₁, ..., kₙ zur Verfügung hält. Wie auch im vorangehenden Beispiel werden die die Arzneimittel 102 enthaltenden Taschen 103 des Arzneimittelblisters 111 nach dem Einlegen der Arzneimittel 102 geschlossen und erst unmittelbar vor der Einnahme des Arzneimittels 102 durch den Patienten 44 geöffnet. Der jeweilige Schlüssel k₁, ..., kₙ der zuletzt geöffneten Tasche 103 wird gemeinsam mit der Kennung m₁, ..., mₙ des jeweiligen Arzneimittelblisters 111 an den zentralen Server übertragen und der zentrale Server übermittelt die auf ihm gespeicherte und dem Schlüssel k₁, ..., kₙ sowie der Kennung m₁, ..., mₙ zugeordnete Wirkstoffmenge c an den Patienten 44 zurück. Anschließend wird der jeweilige Wirkstoffwert c an die Stellgrößen-Speichereinheit 45 übertragen, wobei der jeweilige Wirkstoffwert c gemeinsam mit dem jeweiligen Ausgabezeitpunkt in einem dem Patienten 44 zugeordneten Speicher der Stellgrößen-Speichereinheit 45 abgespeichert wird.

## Patentansprüche

1. Verfahren zur Erstellung und Verpackung und Dokumentation der Entnahme einer Anzahl von Arzneimitteln (102) mit einem vorgegebenen Wirkstoff sowie zur gleichzeitigen Bereitstellung eines die jeweilige im Arzneimittel (102) enthaltene Wirkstoffmenge angebenden Wirkstoffwerts (c),
- wobei für die jeweils festzulegende Wirkstoffmenge der Arzneimittel (102) ein Normwert (c_{N}) und eine maximale Abweichung (Δc) von diesem Normwert (c_{N}) vorgegeben werden, wobei ein Wirkstoffwert (c) innerhalb des Intervalls c_{N} +/- Δc gewählt oder vorgegeben wird und für die Erstellung des jeweiligen Arzneimittels (102) eine Wirkstoffmenge herangezogen wird, die dem Wirkstoffwert (c) entspricht,
- wobei der erstellte Wirkstoffwert (c) dem Arzneimittel (102) zugeordnet und nach der Erstellung des Arzneimittels (102) gemeinsam mit diesem zur Verfügung gehalten wird,
- wobei das erstellte Arzneimittel (102) nach seiner Herstellung in eine vorgegebene Tasche (103) eines Arzneimittelblisters (101), insbesondere eines Arzneimittelblisters, eingelegt wird,
- wobei der dem Arzneimittel (102) zugeordnete Wirkstoffwert (c) sowie ein die vorgegebene jeweilige Tasche (103) des Arzneimittelblisters (101) charakterisierender Schlüssel (k₁, ..., kₙ) gemeinsam an einen, vorzugsweise auf einem auf dem Arzneimittelblister (101) befindlichen Controller (104) befindlichen, Speicher (105) übertragen werden, und
- wobei ein die jeweiligen Tasche (103) charakterisierender Schlüssel (k₁, ..., kₙ) sowie die jeweilige Wirkstoffmenge (c₁, ..., cₙ) des in der jeweiligen Tasche (103) befindlichen Arzneimittels (102), insbesondere vom Controller (104), zum Abruf zur Verfügung gehalten werden,
- wobei die die Arzneimittel (102) enthaltenden Taschen (103) des Arzneimittelblisters (101) nach dem Einlegen der Arzneimittel (102) verschlossen werden, und vor der Einnahme des Arzneimittels (102) die jeweilige Tasche (103) vom Patienten geöffnet wird,
- wobei von dem auf dem Arzneimittelblister (101) befindlichen Controller (104) das Öffnen der jeweiligen Tasche (103) detektiert wird,
- wobei der jeweilige Wirkstoffwert (c) des in der zuletzt geöffneten Tasche (103) befindlichen Arzneimittels (102) separat und/oder zusätzlich, insbesondere vom Controller (104), zur Verfügung gehalten wird, und
- wobei gegebenenfalls der jeweilige Wirkstoffwert (c) an einen Dokumentationsserver übertragen wird, wobei der jeweilige Wirkstoffwert (c) gemeinsam mit dem jeweiligen Ausgabezeitpunkt in einem dem jeweiligen Patienten zugeordneten Dokumentationsspeicher des Dokumentationsservers abgespeichert wird.

2. Verfahren nach Anspruch 1, **dadurch gekennzeichnet, dass** die Wirkstoffwerte (c) der einzelnen Arzneimittel (102) als, insbesondere einer vorgegebenen Verteilung folgend, insbesondere gleichverteilt oder diskret verteilt, Zufallswerte innerhalb des vorgegebenen Intervalls c_{N} +/- Δc vorgegeben werden.

3. Verfahren nach Anspruch 1 oder 2, **dadurch gekennzeichnet, dass** die maximale Abweichung (Δc) vom Normwert (c_{N}) mehr als 5%, insbesondere mehr als 10%, des Normwerts (c_{N}) beträgt.

4. Verfahren nach einem der vorangehenden Ansprüche, **dadurch gekennzeichnet,**
- **dass** eine den Wirkstoff enthaltende Wirkstoffflüssigkeit (121), insbesondere eine Lösung, Emulsion oder Suspension, sowie eine Anzahl von Arzneimittelträgern (120), insbesondere Pulvertabletten, zur Verfügung gestellt werden,
- **dass** eine vorgegebene Menge der Wirkstoffflüssigkeit (121) auf den Arzneimittelträger (120) aufgetropft wird und vom Arzneimittelträgerstoff des Arzneimittelträgers (120) aufgenommen wird, wobei während des Falls des jeweiligen Tropfens (122) in Richtung des Arzneimittelträgers (120) das Volumen des jeweiligen Tropfens (122) bestimmt wird, und
- **dass** für das jeweilige Arzneimittel (102) die jeweilige Größe des Tropfens (122) als Wirkstoffwert (c) dem erstellten Arzneimittel (102) zugeordnet und das Arzneimittel (102) gemeinsam mit dem ihm zugeordneten Wirkstoffwert (c) zur Verfügung gehalten wird.

5. Verfahren nach einem der vorherigen Ansprüche, **dadurch gekennzeichnet,**
- **dass** das erstellte Arzneimittel (102) in eine vorgegebene Tasche (103) eines Arzneimittelblisters (111) eingelegt wird, der einen Controller (114) aufweist, auf dem eine vorgegebene, den Arzneimittelblister (111) kennzeichnende Kennung (m₁, ..., mₙ) abgespeichert ist,
- **dass** die dem Arzneimittel (102) zugeordnete Wirkstoffmenge (c₁, ..., cₙ), die dem jeweiligen Arzneimittelblister (111) zugeordnete Kennung (m₁, ..., mₙ) sowie ein die vorgegebene jeweilige Tasche (103) des Arzneimittelblisters (111) charakterisierender Schlüssel (k₁, ..., kₙ) gemeinsam an einen Server übertragen werden, und
- **dass** der jeweilige Wirkstoffwert (c₁, ..., cₙ) des in der jeweiligen Tasche (103) befindlichen Arzneimittels (102) bei Übermittlung der den Arzneimittelblister (111) charakterisierenden Kennung (m₁, ..., mₙ) sowie des die jeweiligen Tasche (103) charakterisierenden Schlüssels (k₁, ..., kₙ) vom Server zum Abruf zur Verfügung gehalten wird.

6. Verfahren nach Anspruch 5, **dadurch gekennzeichnet,**
- **dass** die die Arzneimittel (102) enthaltenden Taschen (103) des Arzneimittelblisters (111) nach dem Einlegen der Arzneimittel (102) verschlossen werden, und vor der Einnahme des Arzneimittels (102) die jeweilige Tasche (103) vom Patienten geöffnet wird,
- **dass** von dem auf dem Arzneimittelblister (111) befindlichen Controller (114) die jeweils geöffnete Tasche (103) detektiert wird,
- **dass** der jeweilige Schlüssel (k₁, ..., kₙ) der zuletzt geöffneten Tasche (103) gemeinsam mit der Kennung (m) des jeweiligen Arzneimittelblisters an den Server übertragen wird und der Server die auf ihm gespeicherte und dem Schlüssel sowie der Kennung (m) zugeordnete Wirkstoffmenge (c₁, ..., cₙ) ermittelt und zur Verfügung hält, und
- **dass** gegebenenfalls der jeweilige Wirkstoffwert (c) an eine Stellgrößen-Speichereinheit (45) übertragen wird, wobei der jeweilige Wirkstoffwert (c) gemeinsam mit dem jeweiligen Ausgabezeitpunkt in einem dem jeweiligen Patienten zugeordneten Speicher der Stellgrößen-Speichereinheit (45) abgespeichert werden.

7. Kombination umfassend
- zumindest einen Arzneimittelblister (101; 111) umfassend eine Anzahl von Arzneimitteln (102), insbesondere Tabletten oder Kapseln, die in im Arzneimittelblister (101; 111) ausgebildeten, und insbesondere ausschließlich das jeweilige Arzneimittel (102) enthaltenden, Taschen (103) angeordnet sind, wobei die einzelnen Arzneimittel (102) jeweils eine unterschiedliche Wirkstoffmenge (c₁, ..., cₙ) aufweisen, und
- einen Speicher (105), der jeweils für jede der Taschen (103) des Arzneimittelblisters (101; 111) jeweils einen Speicherplatz aufweist, in dem ein Wirkstoffwert (c₁, ..., cₙ) abgespeichert ist, der der Wirkstoffmenge des in der jeweiligen Tasche (103) befindlichen Arzneimittels (102) entspricht, wobei der Speicher (105) auf Anfrage bei Übermittlung einer die Tasche (103) eindeutig kennzeichnenden Kennung (m₁, ..., mₙ) den jeweiligen dem Arzneimittel (102) in der jeweiligen Taschen (103) zugeordneten Wirkstoffwert (c) zur Verfügung hält.

8. Kombination nach Anspruch 7, **dadurch gekennzeichnet, dass** auf dem Arzneimittelblister (101; 111) ein Controller (104; 114) vorgesehen ist,
a) wobei auf dem Controller (104; 114) eine den Arzneimittelblister (101; 111) eindeutig kennzeichnende Kennung (m₁, ..., mₙ) abgespeichert ist und/oder
b) wobei der Controller (104; 114) das Öffnen von auf dem Arzneimittelblister (101; 111) ausgebildeten Tasche (103) überwacht und eine die jeweils zuletzt geöffnete Tasche (103) eindeutig kennzeichnende Kennung (m₁, ..., mₙ) zur Verfügung hält.

9. Kombination nach Anspruch 8, **dadurch gekennzeichnet, dass** der Speicher (105) in dem auf dem Arzneimittelblister (101) angeordneten Controller (104) integriert ist, wobei im Speicher (105) für jedes in den einzelnen Taschen (103) befindliche Arzneimittel (102) jeweils ein Speicherbereich vorgesehen ist, in dem die jeweilige Wirkstoffmenge (c₁, ..., cₙ) des Arzneimittels (102) abgespeichert ist, und
- dass der Controller (104) auf Anfrage bei Angabe eines die jeweiligen Tasche (103) charakterisierenden Schlüssels (k₁, ..., kₙ) die jeweilige Wirkstoffmenge (c₁, ..., cₙ) des in der jeweiligen Tasche (103) befindlichen Arzneimittels (102) angibt.

10. Kombination nach einem der Ansprüche 8 oder 9, **dadurch gekennzeichnet,**
- **dass** der auf dem Arzneimittelblister (111) ausgebildete Controller (114) einen Identifikationsspeicher aufweist, auf dem eine dem Arzneimittelblister (111) eindeutig kennzeichnende Kennung (m₁, ..., mₙ) vorgesehen ist,
- **dass** der Speicher auf einem Server ausgebildet ist, wobei der Server gegebenenfalls eine Anzahl von Speichern aufweist, wobei jeder Speicher jeweils einem Arzneimittelblister (111) zugeordnet ist,
- wobei im jeweiligen für den Arzneimittelblister (111) vorgesehenen Speicher für jedes in den einzelnen Taschen (103) des Arzneimittelblisters (111) befindliche Arzneimittel (102) jeweils ein Speicherbereich vorgesehen ist, in dem der jeweilige Wirkstoffwert (c₁, ..., cₙ) des in der Tasche (103) des Arzneimittelblisters (111) befindlichen Arzneimittels (102) abgespeichert ist, und
- **dass** der Server auf Anfrage bei Angabe der den Arzneimittelblister (111) charakterisierenden Kennung (m₁, ..., mₙ) sowie eines die jeweilige Tasche (103) des Arzneimittelblisters (111) charakterisierenden Schlüssels (k₁, ..., kₙ) die jeweilige Wirkstoffmenge (c₁, ..., cₙ) des in der jeweiligen Tasche (103) befindlichen Arzneimittels (102) übermittelt.

11. Kombination nach einem der Ansprüche 8 bis 10, **dadurch gekennzeichnet, dass** die Wirkstoffwerte (c) der einzelnen Arzneimittel (102) innerhalb des vorgegebenen Intervalls c_{N} +/- Δc liegen, wobei c_{N} einen Normwert darstellt und Δc die maximale gewünschte Abweichung des Wirkstoffwerts (c) von diesem Normwert (c_{N}) darstellt, wobei insbesondere
a) die Wirkstoffwerte (c) der einzelnen Arzneimittel innerhalb des Intervalls c_{N} +/- Δc einer vorgegebenen Verteilung folgen, beispielsweise gleichverteilt oder diskret verteilt sind, und/oder
b) die maximale Abweichung (Δc) vom Normwert (c_{N}) mehr als 5%, insbesondere mehr als 10%, des Normwerts (c_{N}) beträgt.

12. Vorrichtung (130; 140) zur Herstellung von Arzneimitteln umfassend
a) eine Bereitstellungseinheit (131; 141) zur Bereitstellung von Arzneimittelträgern (120), insbesondere Pulvertabletten,
b) eine Portionierungseinheit (132; 142) zur Portionierung des Wirkstoffs, insbesondere einer Wirkstoffflüssigkeit (121), sowie zum Aufbringen des Wirkstoffs, insbesondere zum Auftropfen der Wirkstoffflüssigkeit (121), auf die von der Bereitstellungseinheit (131; 141) bereit gestellten Arzneimittelträger (120),
**gekennzeichnet durch**
c) eine Steuereinheit (133; 143) zur Steuerung der Größe der von der Portionierungseinheit (132; 142) abgegebenen Menge des Wirkstoffs, insbesondere der Tropfen (122), wobei die Steuereinheit (133; 143) für die jeweils festzulegende Wirkstoffmenge der Arzneimittel (102) einen Normwert (c_{N}) festlegt, und die Steuereinheit (133; 143) der Portionierungseinheit (132; 142) jeweils eine aufzubringende Wirkstoffmenge innerhalb eines Intervalls rund um den Normwert (c_{N}) vorgibt,
- wobei die Steuereinheit (133; 143) die vorgegebene oder die im Arzneimittel (102) enthaltene Wirkstoffmenge als Wirkstoffwert (c) des erstellten Arzneimittels (102) nach dessen Erstellung zur Verfügung hält und in einem dafür vorgesehenen Speicher dauerhaft abspeichert.

13. Vorrichtung (130) nach Anspruch 12, **dadurch gekennzeichnet,**
- **dass** die Steuereinheit (133) zur Steuerung der Größe der von der Portionierungseinheit (132) abgegebenen Menge des Wirkstoffs ausgebildet ist,
- **dass** die Steuereinheit (133) für die jeweils festzulegende Wirkstoffmenge der Arzneimittel (102) einen Normwert (c_{N}) und eine maximale Abweichung (Δc) von diesem Normwert, festlegt,
- **dass** die Steuereinheit (133) der Portionierungseinheit (132) jeweils eine aufzubringende Wirkstoffmenge, insbesondere einen Zufallswert (c_{R}) innerhalb des Intervalls c_{N} +/- Δc, als Wirkstoffwert (c) vorgibt, wobei die Steuereinheit (133) den vorgegebenen Wirkstoffwert (c) nach der Erstellung des Arzneimittels zur Verfügung hält.

14. Vorrichtung (140) nach Anspruch 12 oder 13, **dadurch gekennzeichnet,**
- **dass** die Portionierungseinheit (142) zur Portionierung der Wirkstoffflüssigkeit (121) sowie zum Auftropfen der Wirkstoffflüssigkeit (121) auf die von der Bereitstellungseinheit (141) bereit gestellten Arzneimittelträgern (120), ausgebildet ist, und die Positionierungseinheit die Tropfen der Wirkstoffflüssigkeit (121) mit einer vorgegebenen durchschnittlichen Tropfengröße (c_{N}) und einer bekannten maximalen Abweichung (Δc) von der vorgegebenen durchschnittlichen Tropfengröße (c_{N}) abgibt, und
- **dass** die Steuereinheit (143) eine Einheit (144) zur Bestimmung der Tropfengrößen von von der Portionierungseinheit (142) auf die Arzneimittelträger (120) aufgebrachten Tropfen (122) vorgesehen ist, welche die Größe der jeweiligen auf die Arzneimittelträger (120) aufgebrachten Tropfen (122) als Wirkstoffwert (c) zur Verfügung hält.

## Claims

1. A method for producing and packaging and documenting the removal of a plurality of medicaments (102) comprising a predetermined active substance and for simultaneously providing an active substance value (c) indicating an active substance quantity contained in the medicament (102),
- wherein for the active substance quantity, to be determined in each case, of the medicament (102), a standard value (c_{N}) and a maximum deviation (Δc) from said standard value (c_{N}) are predetermined, wherein an active substance value (c) within the range c_{N} +/-Δc is selected or predetermined and an active substance quantity corresponding to the active substance value (c) is used for producing said medicament (102),
- wherein the active substance value (c) produced is associated with the medicament (102) and is kept available together with the medicament (102) after the production of same,
- wherein the produced medicament (102) is inserted, after it has been produced, into a predetermined pocket (103) of a medicament blister (101), in particular of a medicament blister,
- wherein the active substance value (c) associated with the medicament (102) and a key (k₁, ..., kₙ) characterising a predetermined pocket (103) of the medicament blister (101) are transferred together to a memory (105) disposed preferably on a controller (104) which is disposed on the medicament blister (101), and
- wherein a key (k₁, ..., kₙ) characterising a pocket (103) and an active substance quantity (c₁, ..., cₙ) of the medicament (102) disposed in said pocket (103) are kept available for retrieval, in particular by the controller (104),
- wherein the pockets (103) of the medicament blister (101) containing the medicaments (102) are closed after the medicaments (102) are inserted, and a pocket (103) is opened by the patient before the medicament (102) is taken,
- wherein the opening of a pocket (103) is detected by the controller (104) disposed on the medicament blister (101),
- wherein an active substance value (c) of the medicament (102) disposed in the last-opened pocket (103) is kept available separately and/or additionally, in particular by the controller (104), and
- wherein optionally an active substance value (c) is transferred to a documentation server, wherein said active substance value (c) is stored together with an output time in a documentation memory, associated with a patient, of the documentation server.

2. The method according to claim 1, **characterised in that** the active substance values (c) of the individual medicaments (102) are predetermined, in particular following a predetermined distribution, in particularly in an evenly distributed or discretely distributed manner, as random values within the predetermined range c_{N} +/-Δc.

3. The method according to claim 1 or 2, **characterised in that** the maximum deviation (Δc) from the standard value (c_{N}) is more than 5%, in particular more than 10%, of the standard value (c_{N}).

4. The method according to one of the preceding claims, **characterised in**
- **that** an active substance liquid (121), in particular a solution, emulsion or suspension, containing the active substance, and a plurality of excipients (120), in particular powder tablets, are provided,
- **that** a predetermined quantity of the active substance liquid (121) is dripped onto the excipient (120) and absorbed by the excipient substance of the excipient (120), wherein the volume of a drop (122) is determined in the case of said drop (122) in the direction of the excipient (120), and
- in that for a medicament (102) a size of the drop (122) is associated with the medicament (102) produced as an active substance value (c) and the medicament (102) is kept available together with the active substance value (c) associated therewith.

5. The method according to one of the preceding claims, **characterised in**
- **that** the produced medicament (102) is inserted into a predetermined pocket (103) of a medicament blister (111), which comprises a controller (114) on which a predetermined identifier (m₁, ..., mₙ) characterising the medicament blister (111) is stored,
- **that** the active substance quantity (c₁, ..., cₙ) associated with the medicament (102), the identifier (m₁, ..., mₙ) associated with a medicament blister (111) and a key (k₁, ..., kₙ) characterising a predetermined pocket (103) of the medicament blister (111) are transferred together to a server, and
- **that** an active substance value (c₁, ..., cₙ) of the medicament (102) disposed in a pocket (103) is kept available for retrieval when the identifier (m₁, ..., mₙ) characterising the medicament blister (111) and a key (k₁, ..., kₙ) characterising said pocket (103) are transferred from the server.

6. The method according to claim 5, **characterised in**
- **that** the pockets (103), containing the medicaments (102), of the medicament blister (111) are closed after the medicaments (102) are inserted, and a pocket (103) is opened by the patient before the medicament (102) is taken,
- **that** the pocket (103) opened in each case is detected by the controller (114) disposed on the medicament blister (111),
- **that** a key (k₁, ..., kₙ) of the last-opened pocket (103) together with the identifier (m) of a medicament blister is transferred to the server, and the server determines and keeps available the active substance quantity (c₁, ..., cₙ) which is stored thereon and associated with the key and the identifier (m), and
- **that** optionally an active substance value (c) is transferred to a manipulated variable memory unit (45), wherein said active substance value (c) is stored together with an output time in a memory, associated with a patient, of the manipulated variable memory unit (45).

7. A combination comprising
- at least one medicament blister (101; 111) comprising a plurality of medicaments (102), in particular tablets or capsules, which are arranged in pockets (103) which are formed in the medicament blister (101; 111) and contain exclusively said medicament (102), wherein the individual medicaments (102) each have a different active substance quantity (c₁, ..., cₙ), and
- a memory (105) which in each case has a memory space for each of the pockets (103) of the medicament blister (101; 111), in which memory space an active substance value (c₁, ..., cₙ) is stored, which corresponds to the active substance quantity of the medicament blister (102) disposed in a pocket (103), wherein the memory (105), on request when transferring an identifier (m₁, ..., mₙ) that uniquely characterises the pocket (103), keeps the active substance value (c) available which is associated with a medicament (102) in a pocket (103).

8. The combination according to claim 7, **characterised in that** a controller (104; 114) is provided on the medicament blister (101; 111),
a) wherein an identifier (m₁, ..., mₙ) uniquely characterising the medicament blister (101; 111) is stored on the controller (104; 114) and/or
b) wherein the controller (104; 114) monitors the opening of the pocket (103) formed on the medicament blister (101; 111) and keeps an identifier (m₁, ..., mₙ) available which uniquely characterises the last-opened pocket (103).

9. The combination according to claim 8, **characterised in that** the memory (105) is integrated in the controller (104) arranged on the medicament blister (101), wherein in the memory (105) a memory region, in which an active substance quantity (c₁, ..., cₙ) of the medicament (102) is stored, is provided in each case for each medicament (102) disposed in the individual pockets (103), and
- that the controller (104), on request when a key (k₁, ..., kₙ) characterising a pocket (103) is specified, specifies an active substance quantity (c₁, ..., cₙ) of the medicament (102) disposed in the pocket (103).

10. The combination according to one of claims 8 or 9, **characterised in**
- **that** the controller (114) formed on the medicament blister (111) has an identification memory on which an identifier (m₁, ..., mₙ) uniquely characterising the medicament blister (111) is provided,
- **that** the memory is formed on a server, wherein the server optionally has a plurality of memories, wherein each memory is associated in each case with a medicament blister (111),
- wherein in a memory provided for the medicament blister (111), a memory region, in which an active substance value (c₁, ..., cₙ) of the medicament (102) disposed in the pocket (103) of the medicament blister (111) is stored, is provided for each medicament (102) disposed in the individual pockets (103) of the medicament blister (111), and
- **that** the server, on request when the identifier (m₁, ..., mₙ) characterising the medicament blister (111) and a key (k₁, ..., kₙ) characterising a pocket (103) of the medicament blister (111) are specified, specifies an active substance quantity (c₁, ..., cₙ) of the medicament (102) disposed in the pocket (103).

11. The combination according to one of claims 8 to 10, **characterised in that** the active substance values (c) of the individual medicaments (102) lie within the predetermined range c_{N} +/- Δc, wherein c_{N} constitutes a standard value and Δc constitutes the maximum desired deviation of the active substance value (c) from said standard value (c_{N}), wherein in particular
a) the active substance values (c) of the individual medicaments within the range c_{N} +/Δc follow a predetermined distribution, for example in an evenly distributed or discretely distributed manner, and/or
b) the maximum deviation (Δc) from the standard value (c_{N}) is more than 5%, in particular more than 10%, of the standard value (c_{N}).

12. A device (130; 140) for producing medicaments, comprising
a) a supply unit (131; 141) for providing excipients (120), in particular powder tablets,
b) a portioning unit (132; 142) for portioning the active substance, in particular an active substance liquid (121), and for applying the active substance, in particular for dripping the active substance liquid (121), onto the excipients (120) supplied by the supply unit (131; 141),
**characterised by**
c) a control unit (133; 143) for controlling the size of the quantity of active substance, in particular of drops (122), output by the portioning unit (132; 142), wherein the control unit (133; 143) establishes a standard value (c_{N}) for the active substance quantity, to be determined in each case, of the medicament (102), and the control unit (133; 143) of the portioning unit (132; 142) in each case specifies an active substance quantity to be applied within a range around the standard value (c_{N}),
- wherein the control unit (133; 143) keeps the active substance quantity predetermined or contained in the medicament (102) available as an active substance value (c) of the medicament (102) produced after same is produced and permanently stores said active substance quantity in a memory provided therefor.

13. The device (130) according to claim 12, **characterised in**
- **that** the control unit (133) is designed to control the quantity of the active substance output by the portioning unit (132),
- **that** the control unit (133) for the active substance quantity, to be determined in each case, of the medicaments (102) establishes a standard value (c_{N}) and a maximum deviation (Δc) from said standard value,
- **that** the control unit (133) of the portioning unit (132) in each case predetermines an active substance quantity to be applied, in particular a random value (c_{R}) within the range c_{N} +/- Δc, as an active substance value (c), wherein the control unit (133) keeps the predetermined active substance value (c) available after the medicament has been produced.

14. The device (140) according to claim 12 or 13, **characterised in**
- **that** the portioning unit (142) for portioning the active substance liquid (121) and for dripping the active substance liquid (121) onto the excipients (120) supplied by the supply unit (141), and the positioning unit outputs the drops of the active substance liquid (121) with a predetermined average drop size (c_{N}) and a known maximum deviation (Δc) from the predetermined average drop size (c_{N}), and
- **that** the control unit (143) provides a unit (144) for determining the drop sizes, from the portioning unit (142), of the drops (122) applied to the excipients (120), said unit keeping the sizes of the respective drops (122) applied to the medicament carriers (120) available as active substance values (c).

## Revendications

1. Procédé de production et emballage et documentation de prélèvement d'un certain nombre de médicaments (102) avec un principe actif prédéfini, ainsi que de fourniture simultanée d'une valeur de principe actif (c) indiquant la quantité de principe actif contenue respectivement dans le médicament (102),
- dans lequel, pour la quantité de principe actif à fixer du médicament (102), une valeur de norme (c_{N}) et un écart maximum (Δc) par rapport à cette valeur de norme (c_{N}) sont prédéfinis, dans lequel une valeur de principe actif (c) est choisie ou prédéfinie au sein de l'intervalle c_{N} +/- Δc et, pour la production du médicament respectif (102), une quantité de principe actif correspondant à la valeur de principe actif (c) est établie,
- dans lequel la valeur de principe actif (c) produite est attribuée au médicament (102) et, après la production du médicament (102), conservée avec celui-ci pour mise à disposition,
- dans lequel le médicament (102) produit, après sa production, est placé dans une poche prédéfinie (103) d'un emballage-coque pour médicaments (101), en particulier un emballage-coque pour médicaments,
- dans lequel la valeur de principe actif (c) attribuée au médicament (102), ainsi qu'une clé de caractérisation (k₁, ..., kₙ) de la poche prédéfinie respective (103) de l'emballage-coque pour médicaments (101) sont transmises ensemble à une mémoire (105) se trouvant de préférence sur un organe de commande (104) se trouvant sur l'emballage-coque pour médicaments (101) et
- dans lequel une clé de caractérisation (k₁, ..., kₙ) de la poche respective (103), ainsi que la quantité de principe actif respective (c₁, ..., cₙ) du médicament (102) se trouvant dans la poche respective (103), sont conservées en particulier par l'organe de commande (104), pour mise à disposition sur demande,
- dans lequel les poches (103) contenant les médicaments (102) de l'emballage-coque pour médicaments (101) sont fermées après introduction du médicament (102) et la poche respective (103) est ouverte par les patients avant le prélèvement du médicament (102),
- dans lequel l'organe de commande (104) se trouvant sur l'emballage-coque pour médicaments (101) détecte l'ouverture de la poche respective (103),
- dans lequel la valeur de principe actif (c) du médicament (102) se trouvant dans la dernière poche ouverte (103) est conservée pour mise à disposition séparément et/ou en plus, en particulier par l'organe de commande (104) et
- dans lequel, le cas échéant, la valeur de principe actif respective (c) est transmise à un serveur de documentation, la valeur de principe actif respective (c) étant stockée conjointement avec l'heure de sortie respective, dans une mémoire de documentation attribuée au patient respectif du serveur de documentation.

2. Procédé selon la revendication 1, **caractérisé en ce que** les valeurs de principe actif (c) des médicaments individuels (102) sont fournies au préalable comme une valeur aléatoire, suivant en particulier une répartition prédéfinie, notamment réparties également ou de manière discrète au sein de l'intervalle prédéfini c_{N} +/- Δc.

3. Procédé selon la revendication 1 ou 2, **caractérisé en ce que** l'écart maximum (Δc) par rapport à la valeur de norme (c_{N}) est supérieur à 5 %, en particulier supérieur à 10 %, de la valeur de norme (c_{N}).

4. Procédé selon une des revendications précédentes, **caractérisé en ce**
- **qu'**un fluide de principe actif contenant le principe actif (121), notamment une solution, une émulsion ou une suspension, ainsi qu'un certain nombre d'excipients (120) de médicaments, notamment des comprimés de poudre, sont mis à disposition,
- en ce qu'une quantité prédéfinie du fluide de principe actif (121) est déposée par gouttes sur l'excipient (120) de médicament et absorbée par le matériau d'excipient de l'excipient (120) du médicament, dans lequel, pendant la chute des gouttes respectives (122) en direction de l'excipient (120) de médicament, le volume des gouttes respectives (122) est déterminé et
- en ce que, pour le médicament respectif (102), la grandeur respective des gouttes (122) est attribuée comme valeur de principe actif (c) au médicament produit (102) et le médicament (102) est conservée conjointement avec la valeur de principe actif (c) qui lui est attribuée pour mise à disposition.

5. Procédé selon une des revendications précédentes, **caractérisé en ce**
- **que** le médicament produit (102) est inséré dans une poche prédéfinie (103) d'un emballage-coque pour médicaments (111), qui présente un organe de commande (114), sur lequel un identifiant (m₁, ..., mₙ) caractérisant l'emballage-coque pour médicaments (111) est mémorisé,
- en ce que la quantité de principe actif (c₁, ..., cₙ) associée au médicament (102), l'identifiant (m₁, ..., mₙ) associé à l'emballage-coque pour médicaments respectif (111), ainsi qu'une clé (k₁, ..., kₙ) caractérisant la poche prédéfinie respective (103) de l'emballage-coque pour médicaments (111) sont transmis conjointement à un serveur et
- en ce que la valeur de principe actif respective (c₁, ..., cₙ) du médicament (102) se trouvant dans la poche respective (103) est conservée pour mise à disposition sur demande lors de la transmission de l'identifiant (m₁, ..., mₙ) caractérisant l'emballage-coque pour médicaments (111), ainsi que la clé (k₁, ..., kₙ) caractérisant la poche respective (103) par le serveur.

6. Procédé selon la revendication 5, **caractérisé en ce**
- **que** les poches (103) contenant les médicaments (102) de l'emballage-coque pour médicaments (111) sont fermées après introduction du médicament (102) et la poche respective (103) est ouverte par les patients avant le prélèvement du médicament (102),
- en ce que l'organe de commande (114) se trouvant sur l'emballage-coque pour médicaments (111) détecte l'ouverture de la poche respective (103),
- en ce que la clé respective (k₁, ..., kₙ) de la poche ouverte en dernier (103) est transmise conjointement avec l'identifiant (m) de l'emballage-coque respectif au serveur et le serveur conserve pour mise à disposition la quantité de principe actif (c₁, ..., cₙ) associée à la clé et à l'identifiant (m) et mémorisée sur lui et
- en ce que, le cas échéant, la valeur de principe actif respective (c) est transmise à une unité de mémoire de grandeurs de consigne (45), la valeur de principe actif respective (c) étant stockée conjointement avec l'heure de sortie respective, dans une mémoire de documentation attribuée au patient respectif de l'unité de mémoire de grandeurs de consigne (45).

7. Combinaison comprenant
- au moins un emballage-coque pour médicaments (101 ; 111) comprenant un certain nombre de médicaments (102), en particulier des comprimés ou capsules, disposés dans les poches (103) formées dans l'emballage-coque pour médicaments (101 ; 111) et contenant en particulier exclusivement le médicament respectif (102), dans laquelle les médicaments individuels (102) présentent respectivement une quantité de principe actif (c₁, ..., cₙ) différente et
- une mémoire (105), qui présente respectivement pour chacune des poches (103) de l'emballage-coque pour médicaments (101 ; 111) un emplacement de mémoire, dans lequel une valeur de principe actif (c₁, ..., cₙ) est mémorisée, qui correspond à la quantité de principe actif du médicament (102) se trouvant dans la poche respective (103), dans laquelle la mémoire (105) conserve pour mise à disposition sur demande, lors de la transmission d'un identifiant (m₁, ..., mₙ) caractérisant de manière claire la poche (103), la valeur de principe actif (c) attribuée au médicament respectif (102) dans la poche respective (103).

8. Combinaison selon la revendication 7, **caractérisée en ce qu'**un organe de commande (104 ; 114) est prévu sur l'emballage-coque pour médicaments (101 ; 111), a) un identifiant (m₁, ..., mₙ) caractérisant de manière claire l'emballage-coque pour médicaments (101 ; 111) est mémorisé sur l'organe de commande (104 ; 114) et/ou b) dans laquelle l'organe de commande (104 ; 114) surveille l'ouverture de la poche (103) formée sur l'emballage-coque pour médicaments (101 ; 111) et tient à disposition un identifiant (m₁, ..., mₙ) caractérisant de manière claire la poche (103) ouverte en dernier.

9. Combinaison selon la revendication 8, **caractérisée en ce que** la mémoire (105) est intégrée dans un organe de commande (104) disposé sur l'emballage-coque pour médicaments (101), dans laquelle, pour chaque médicament (102) se trouvant dans les poches (103) individuelles, une zone de mémoire est prévue respectivement dans la mémoire (105), dans laquelle la quantité de principe actif respective (c₁, ..., cₙ) du médicament (102) est mémorisée et
- **en ce que** l'organe de commande (104) indique, sur demande, lors de l'indication d'une clé (k₁, ..., kₙ) caractérisant la poche respective (103), la quantité de principe actif respective (c₁, ..., cₙ) du médicament (102) se trouvant dans la poche respective (103).

10. Combinaison selon une des revendications 8 ou 9, **caractérisé en ce**
- **que** l'organe de commande (114) formé sur l'emballage-coque pour médicaments (111) présente une mémoire d'identification, sur laquelle un identifiant (m₁, ..., mₙ) caractérisant de manière unique l'emballage-coque pour médicaments (111) est prévu,
- en ce que la mémoire est formée sur un serveur, dans laquelle le serveur présente le cas échéant un certain nombre de mémoires, dans laquelle chaque mémoire est attribuée respectivement à un emballage-coque pour médicaments (111),
- dans laquelle, dans les mémoires prévues respectivement pour les emballages-coques pour médicaments (111), une zone de mémoire est prévue respectivement pour chaque médicament (102) se trouvant dans les poches respectives (103) de l'emballage-coque pour médicaments (111), zone dans laquelle la valeur de principe actif respective (c₁, ..., cₙ) du médicament (102) se trouvant dans la poche (103) de l'emballage-coque pour médicaments (111) est stockée et
- en ce que le serveur, sur demande, lors de l'indication de l'identifiant (m₁, ..., mₙ) caractérisant l'emballage-coque pour médicaments (111) ainsi que d'une clé (k₁, ..., kₙ) caractérisant la poche respective (103) de l'emballage-coque pour médicaments (111), transmet la quantité de principe actif respective (c₁, ..., cₙ) du médicament (102) se trouvant dans la poche respective (103).

11. Combinaison selon une des revendications 8 à 10, **caractérisée en ce que** les valeurs de principe actif (c) des médicaments (102) individuels se trouvent au sein de l'intervalle prédéfini c_{N} +/- Δc, dans laquelle c_{N} représente une valeur de norme et Δc représente l'écart maximum souhaité de la valeur de principe actif (c) par rapport à cette valeur de norme (c_{N}), dans laquelle notamment
a) les valeurs de principe actif (c) des médicaments individuels présentent une répartition prédéfinie au sein de l'intervalle c_{N} +/- Δc, par exemple sont réparties également ou de manière discrète et/ou
b) l'écart maximum (Δc) par rapport à la valeur de norme (c_{N}) est supérieur à 5 %, en particulier supérieur à 10 %, de la valeur de norme (c_{N}).

12. Dispositif (130 ; 140) de production de médicaments comprenant
a) une unité de fourniture (131 ; 141) pour la fourniture d'excipients (120) de médicaments, en particulier de comprimés de poudre,
b) une unité de division en portions (132 ; 142) pour la division en portions du principe actif, en particulier d'un fluide de principe actif (121), ainsi que pour l'application du principe actif, en particulier pour l'application par gouttes du fluide de principe actif (121), sur l'excipient (120) de médicament déjà mis à disposition par l'unité de fourniture (131 ; 141),
**caractérisé en ce**
c) qu'une unité de commande (133 ; 143) pour la commande de la grandeur de la quantité du principe actif fournie par l'unité de division en portions (132 ; 142), en particulier des gouttes (122), dans lequel l'unité de commande (133 ; 143) détermine, pour la quantité de principe actif respective à fixer du médicament (102), une valeur de norme (c_{N}) et l'unité de commande (133 ; 143) indique à l'unité de division en portions (132 ; 142) respectivement une quantité de principe actif à appliquer au sein d'un intervalle autour de la valeur de norme (c_{N}),
- dans lequel l'unité de commande (133 ; 143) conserve pour mise à disposition la quantité de principe actif prédéfinie ou contenue dans le médicament (102) en tant que valeur de principe actif (c) du médicament produit (102) après sa production et la mémorise de manière durable dans une mémoire prévue à cet effet.

13. Dispositif (130) selon la revendication 12, **caractérisé en ce**
- **que** l'unité de commande (133) est conçue pour la commande de la grandeur de la quantité de principe actif fournie par l'unité de division en portions (132),
- en ce que l'unité de commande (133) détermine, pour les quantités respectives à fixer du médicament (102), une valeur de norme (c_{N}) et un écart maximum (Δc) par rapport à cette valeur de norme,
- en ce que l'unité de commande (133) indique à l'unité de division en portions (132) respectivement une quantité de principe actif à appliquer, en particulier une valeur aléatoire (c_{R}) au sein de l'intervalle c_{N} +/- Δc, comme valeur de principe actif (c), dans lequel l'unité de commande (133) conserve pour mise à disposition la valeur de principe actif prédéfinie (c) après la production du médicament.

14. Dispositif (140) selon la revendication 12 ou 13, **caractérisé en ce**
- **que** l'unité de division en portions (142) est conçue pour la division en portions du fluide de principe actif (121) ainsi que pour l'application par gouttes du fluide de principe actif (121) sur les excipients (120) de médicament déjà fournis par l'unité de fourniture (141) et l'unité de division en portions fournit les gouttes du fluide de principe actif (121) avec une grandeur de goutte moyenne prédéfinie (c_{N}) et un écart maximum connu (Δc) par rapport à la grandeur de goutte moyenne prédéfinie (c_{N}) et
- en ce que l'unité de commande (143) comprend une unité (144) pour la détermination de la grandeur de goutte des gouttes (122) provenant de l'unité de division en portions (142) appliquées sur l'excipient (120) de médicament, qui conserve pour mise à disposition la grandeur des gouttes (122) respectives appliquées sur l'excipient (120) de médicament en tant que valeur de principe actif (c).
